# DEMANDE DE BREVET EUROPEEN

(11) **EP 2 194 132 A1**
(43) Date de publication de la demande: **09.06.2010**
(21) Numéro de dépôt: 10151700.1
(22) Date de dépôt: 14.02.1997
(51) Int. Cl.: C12N 15/30, C12N 15/86, C07K 14/445, C07K 16/20, A61K 39/015

(54) **Composition vaccinale comprenant un mélange de protéine recombinante contenant un fragment C-terminal p19 de la protéine MSP-1 de plasmodium falciparum et une autre proteine recombinante p19 provenant d'un plasmodium homologue à P. falciparum**

(30) Priorité: 14.02.1996 FR 9601822
(62) Demande divisionnaire de: 97905212.3
(71) Demandeur: INSTITUT PASTEUR, 75724 Paris Cedex 15 (FR); NEW YORK UNIVERSITY, New York, NY 10016 (US)
(72) Inventeur: Longacre-Andre, Shirley, 75006 Paris (FR); Roth, Charles, 92500 Rueil Malmaison (FR); Nato, Faridabano, 92160 Antony (FR); Barnwell, John W., New York, NY 10012 (US); Mendis, Kamini, 8 Colombo (LK)
(74) Mandataire: Paris, Fabienne

(57) **Abrégé**

La demande concerne une composition de vaccin comprenant à titre de principes actifs un mélange de:
**a)** une protéine recombinante contenant:
- un fragment C-terminal de 19 kilodaltons (p19) de la protéine 1 de surface (protéine MSP-1) de la forme mérozoïte d'un parasite du type *Plasmodium falciparum*, ce fragment C-terminal restant normalement ancré à la surface du parasite au terme de sa phase de pénétration dans des érythrocytes humains, à l'occasion d'un cycle infectieux; ou
- une partie du dit fragment p19 qui est apte à induire une réponse immune capable d'inhibiter une parasitémie in vivo due à un parasite *Plasmodium falciparum* et qui contient au moins l'une des deux régions EGF contenues dans le dit fragment p19
la dite protéine
- comportant des épitopes conformationnels instables en milieu réducteur et constituant la majorité des épitopes reconnus par des antisérums humains formés contre le *Plasmodium falciparum* et
- étant reconnue par des antisérums humains formés contre *Plasmodium falciparum* lorsqu'elle est à l'état réduit non irréversible, mais non reconnue ou peu reconnue par ces mêmes antisérums lorsqu'elle est irréversiblement réduite,
**et** de
**b)** une autre protéine recombinante de type p19 provenant d'un parasite Plasmodium homologue à *Plasmodium falciparum*.

## Description

L'invention concerne de nouveaux principes actifs de vaccins dérivés de la protéine majeure de surface de formes mérozoïtes d'un *Plasmodium* infectieux pour des mammifères, notamment l'homme, plus généralement connue sous la désignation MSP-1.

Cette protéine a déjà fait l'objet d'études nombreuses. Elle est synthétisée dans le stade schizonte des parasites du type *Plasmodium,* notamment *Plasmodium falciparum,* et est exprimée sous forme de l'un des constituants majeurs de la surface des mérozoïtes aussi bien pendant le stade hépatique que pendant le stade érythrocytaire du paludisme (1, 2, 3, 4). En raison du caractère prédominant et de la conservation dans toutes les espèces de *Plasmodium* connues de cette protéine, il a été suggéré qu'elle pourrait représenter un candidat pour la constitution de vaccins anti-paludiques (5, 6).

Il en a encore été de même pour des fragments de cette protéine, particulièrement des produits naturels de clivage dont l'on observe la formation, par exemple au cours de l'invasion par le parasite des érythrocytes de l'hôte infecté. Parmi, ces produits de clivage, on relèvera le fragment C-terminal ayant un poids moléculaire de 42 kDa (7,8) qui est à son tour clivé une nouvelle fois en un fragment N-terminal ayant un poids moléculaire apparent conventionnel de 33 kDa et en un fragment C-terminal ayant un poids moléculaire apparent conventionnel de 19 kDa (9) qui reste normalement fixé à la membrane du parasite au terme des modifications dont il est lui-même l'objet, par l'intermédiaire de groupes du type glycosylphosphatidylinositol (GPI) (10, 11).

On le retrouve encore au stade anneau précoce du cycle de développement intraérythrocytaire (15, 16), d'où les observations qui ont été faites que ce fragment de 19 kDa pourrait jouer un rôle non encore connu, mais sans doute essentiel dans les processus réinvasifs. De là découlent les hypothèses déjà formulées dans le passé que cette protéine pourrait constituer une cible particulièrement efficace pour d'éventuels vaccins.

Il sera entendu que les références souvent faites dans ce qui suit à des protéines p42 et p19 issues d'un certain type de *Plasmodium* s'entendent comme se rapportant aux produits de clivage C-terminaux correspondants de la protéine MSP-1 de ce *Plasmodium,* ou, par extension, à des produits contenant sensiblement les mêmes séquences en acides aminés, obtenus par recombinaison génétique ou par synthèse chimique selon les techniques classiques, par exemple par synthétiseur de type « Applied System » ou par synthèse sur phase solide de type « Merrifield ». Pour la commodité du langage, les références à des « p42 recombinantes » et des « p19 recombinantes » se rapportent à des « p42 » et « p19 » obtenues par des techniques comportant au moins une étape de génie génétique.

Devant la difficulté d'obtenir des quantités importantes de parasites pour *P.falciparum* et l'impossibilité de cultiver *P.vivax in vitro,* il est devenu évident que le seul moyen de produire un vaccin antipaludique nécessite un recours aux techniques permettant l'utilisation des peptides ou protéines recombinantes. Mais, le MSP-1 est très difficile à produire en entier à cause de sa grande taille d'environ 200 kDa, un fait qui a conduit les chercheurs à s'intéresser à la partie C-terminale dont la fonction, encore inconnue, est vraisemblablement la plus importante.

Au titre, des protéines recombinantes concernant la partie C-terminale de MSP-1 de *P.falciparum*, qui ont été produites et testées chez le singe (12, 40, 41) on mentionnera :
· une p19 fusionnée avec une glutathione-S-transférase produite dans *E. coli* (40),
· une p42 fusionnée avec une glutathione-S-transférase produite dans *E. coli* (12),
· une p19 fusionnée avec un polypeptide issu d'une anatoxine tétanique et porteur d'épitopes de cellules T auxiliaires produites dans *S.cerevisiae* (12),
· une p42 produite dans un système baculovirus (41).

Une composition contenant la protéine de fusion p19 avec une glutathione-S-transférase produite dans *E.coli* en association avec alun ou liposomes n'a pas exercé d'effet protecteur sur aucun des six singes *Aotus nancymai* vaccinés (40)

Une composition contenant la protéine de fusion p42 avec une glutathione-S-transférase produite dans *E.coli* en association avec un adjuvant complet de Freund n'ont pas exercé d'effet protecteur dans les deux types de singes *Aotus (A.nancymai* et *A.vociferans*) auxquels elles avaient été administrées. La protéine p19 produite dans *S.cerevisiae* a exercé un effet protecteur dans deux singes Aotus de type *A.nancymai* (12). Par contre elle n'a pas exercé d'effet protecteur dans deux singes *Aotus* de type *A.vociferans.*

Certains chercheurs (Chang et al.) ont également rapporté des essais d'immunisation réalisés chez le lapin avec une protéine recombinante p42 produite dans un système baculovirus et contenant une séquence d'acides aminés en commun avec *P.falciparum* (18). Ainsi ces derniers auteurs indiquent-ils que cette p42 recombinante se comporte chez le lapin sensiblement de la même façon que la protéine MSP-1 recombinante entière (gp195) Cette protéine p42 en association avec un adjuvant complet de Freund a fait l'objet d'un essai de vaccination dans un primate non-humain susceptible à l'infection par *P.falciparum., Aotus, lemurinus grisemembra* (40). Les résultats montrent que 2 sur 3 animaux ont été complètement protégés et le troisième, bien que montrant une parasitémie semblable aux contrôles, avait une période latente plus longue. Il est néanmoins hasardeux de conclure au caractère protecteur chez l'homme des anticorps ainsi induits à l'encontre des parasites eux-mêmes. Rappelons en effet qu'il n'existe pas actuellement de modèles expérimentaux très satisfaisants chez le primate pour *P. vivax* et *P.falciparum.* Le modèle *Saimiri,* qui a été développé pour *P.falciparum* et *P.vivax,* et le modèle *Aotus* pour *P.falciparum,* sont des systèmes artificiels, nécessitant l'adaptation de souches de parasite et souvent la splénectomie des animaux pour obtenir des parasitémies significatives. En conséquence, les résultats de vaccination provenant de ces modèles ne peuvent avoir qu'une valeur prédictive limitée pour l'Homme.

On peut de toute façon aussi s'interroger sur ce que serait un taux réel de vaccination susceptible d'être éventuellement obtenu avec de telles protéines recombinantes, compte tenu de la constatation -rapportée plus loin- qui a été faite de la présence dans les p42 issues des *Plasmodiums* de la même espèce, et plus particulièrement dans les p33 correspondantes, de régions hypervariables qui rendraient aléatoires en de nombreux cas l'efficacité immunoprotectrice des anticorps induits chez des personnes vaccinées par une p42 issue d'une souche de *Plasmodium* à l'encontre d'une infection par d'autres souches de la même espèce (13).

On peut même supposer que le polymorphisme important de la partie N-terminale du p42 joue un rôle significatif dans l'échappement immunitaire, souvent observé pour ce type de parasites.

La présente invention a pour objectif la production de protéines recombinantes vaccinantes échappant à ces difficultés, dont l'effet protecteur est vérifiable dans des modèles expérimentaux réellement significatifs, ou même directement chez l'homme.

L'invention concerne plus particulièrement des compositions vaccinantes contre un parasite du type *Plasmodium* infectieux pour l'homme, contenant à titre de principe actif une protéine recombinante glycosylée ou non, dont la séquence polypeptidique constitutive essentielle est celle :
- soit d'un fragment C-terminal de 19 kilodaltons (p19) de la protéine 1 de surface (protéine MSP-1) de la forme mérozoïte d'un parasite du type *Plasmodium* infectieux pour l'Homme, ce fragment C-terminal restant normalement ancré à la surface du parasite au terme de sa phase de pénétration dans des érythrocytes humains, à l'occasion d'un cycle infectieux
- soit d'une partie de ce fragment dès lors qu'elle est apte aussi à induire une réponse immune capable d'inhiber une parasitémie in vivo due au parasite correspondant ;
- soit d'un peptide immunologiquement équivalent à ce fragment p19 ou à la susdite partie de ce fragment, et
   cette protéine recombinante comportant en outre des épitopes conformationnels instables en milieu réducteur et constituant, de préférence, la majorité des épitopes reconnus par des antisérums humains formés contre le *Plasmodium* correspondant.

La présence de ces épitopes conformationnels pourrait jouer un rôle important dans l'efficacité protectrice du principe actif de vaccins. On les retrouve tout particulièrement dans les principes actifs présentant par ailleurs les autres caractéristiques définies ci-dessus, lorsqu'ils ont été produits dans un système vecteur baculovirus. S'il en est besoin, il est mentionné ci-après que par l'expression « système de vecteur baculovirus » on entend l'ensemble que constitue le vecteur type baculovirus lui-même et les lignées cellulaires, notamment cellules d'insectes transfectables par un baculovirus modifié par une séquence à transférer dans ces lignées cellulaires avec pour résultat l'expression de cette séquence transférée Des exemples préférés de ces deux partenaires du système baculovirus, ont été décrits dans l'article de Longacre et al. (19) C'est le même système qui a été utilisé dans les exemples qui suivent. Il va naturellement de soi que des variants, tant du baculovirus que des cellules infectables par ce baculovirus peuvent être utilisés en lieu et place de celui qui a été choisi.

En particulier ladite protéine recombinante est reconnue par des antisérums humains formés contre le *Plasmodium* correspondant ou contre un *Plasmodium* homologue lorsqu'elle est à l'état non réduit ou dans un état réduit non irréversible, mais non reconnue ou peu reconnue par ces mêmes antisérums, lorsqu'elle est irréversiblement réduite.

Le caractère instable en milieu réducteur de ces épitopes conformationnels peut être mis en évidence, notamment par le test décrit plus loin dans les exemples, notamment en présence de β-mercaptoéthanol. De même sont indiquées dans des exemples ci-après des conditions expérimentales applicables à l'obtention d'une réduction irréversible des protéines conformes à l'invention.

De ce point de vue, la protéine recombinante produite par S. Longacre et al. (14) peut être mise en oeuvre dans de tels compositions. Il est rappelé que S. Longacre et al. réussirent à produire une p19 recombinante issue de la MSP-1 de *P. vivax* dans un système vecteur à baculovirus contenant une séquence nucléotidique codant pour la p19 de *Plasmodium vivax,* en particulier par transfection de cultures de cellules d'insectes [lignée de *Spodoptera frugiperda* (*Sf*9)] avec des baculovirus vecteurs contenant, sous le contrôle du promoteur de la polyhédrine, une séquence codant pour les fragments peptidiques définis ci-dessous, dont les séquences étaient placées dans l'ordre suivant dans le vecteur baculovirus utilisé :
- fragment 5'-terminal de 35 paires de bases de la séquence signal de la polyhédrine, dont avait été muté (en ATT) le codon méthionine d'initiation de l'expression de cette protéine;
- un fragment 5'-terminal nucléotidique codant pour un peptide de 32 acides aminés correspondant à la partie N-terminale de MSP-1, y compris le peptide signal de MSP-1 ;
- soit une séquence nucléotidique codant pour la p19, soit une séquence codant pour la p42 de la protéine MSP-1 de *Plasmodium vivax*, ces séquences étant aussi, selon le cas, soit pourvues (formes « ancrées »), soit dépourvues (formes solubles) des régions d'extrémité 3' de ces séquences de nucléotides dont les produits d'expression C-terminaux extrêmes sont réputés jouer un rôle essentiel dans l'ancrage de la protéine finale p19 sur la membrane du parasite ;
- 2 codons stop TAA

Pour la p42, les séquences dérivées de la région C-terminale de MSP-1 s'étendaient par conséquent de l'acide aminé Asp 1325 à l'acide aminé Leu 1726 (forme ancrée) ou à l'acide aminé Ser 1705 (forme soluble) et pour la p19, les séquences s'étendaient de l'acide aminé Ile 1602 à l'acide aminé Leu 1726 (forme ancrée) ou à l'acide aminé Ser 1705 (forme soluble), étant entendu que les séquences en acides aminés complètes de p42 et p19 dont les acides aminés initiaux et terminaux ont été indiqués dans ce qui précède découlent du gène de l'isolat Belem de *P.vivax* qui a été séquencé (20).

Des résultats semblables ont été obtenus en mettant en oeuvre dans les mêmes systèmes de vecteurs des séquences nucléotidiques codant pour la p42 et la p19 de *Plasmodium cynomolgi. P.cynomolgi* présente un double intérêt : c'est une espèce parasitaire très proche de *P.vivax* qui est infectieuse pour le macaque. Il peut aussi infecter l'Homme On a également accès à des hôtes naturels de *P.cynomolgi,* les singes rhésus et les singes toques, pour tester l'efficacité de protection du MSP-1 de *P.cynomolgi* dans des systèmes naturels. Le singe rhésus est considéré comme une des espèces les plus représentatives des réactions immunitaires chez l'Homme.

En particulier, on a obtenu d'excellents résultats dans des essais de vaccination réalisés chez le singe toque avec deux polypeptides recombinants la p42 et, surtout, la p19 solubles, dérivées de *P.cynomolgi,* respectivement produites dans un système baculovirus et purifiées sur colonne d'affinité avec des anticorps monoclonaux reconnaissant les régions correspondantes de la protéine MSP-1 native. Les observations suivantes ont été faites : les six singes immunisés avec le seul p19 (trois singes) et le 19 et p42 ensemble (3 singes) ont tous témoigné d'une immunité presque stérile après infection d'épreuve. Les résultats obtenus chez les trois singes immunisés avec le p42 ont été moins significatifs. Deux d'entre eux se sont comportés comme les précédents, mais si le troisième a manifesté une parasitémie moins importante que des témoins immunisés avec un tampon PBS en présence de l'adjuvant de Freund (3 singes) ou non immunisés (3 singes), elle n'en n'était pas moins patente.

Une deuxième infection d'épreuve montrant que les singes ayant reçu la p19 seule étaient protégés au moins pendant six mois. Un deuxième essai de vaccination avec la p19 en association avec alun dans ce système (*P.cynomolgi*/singe toque) a démontré une protection significative pour 2 des 3 singes. C'est la première fois que la MSP-1 ou un autre antigène recombinant a démontré un effet protecteur en présence d'alun (42).

Les résultats des essais particulièrement efficaces réalisés chez le macaque avec des polypeptides recombinants produits dans un système baculovirus mettant en oeuvre une p19 recombinante de *P.cynomolgi,* établissent que des polypeptides recombinants contenant respectivement des p19 recombinantes issus d'autres *Plasmodiums* doivent se comporter de la même manière. Ils sont « plus parlants » pour le paludisme chez l'Homme que les résultats d'essais réalisés avec *P.vivax* ou *P.falciparum* dans leurs « hôtes artificiels ».

Les protéines recombinantes de baculovirus, dérivées d'une partie C-terminale de MSP-1 (p19), ont un effet protecteur antipaludique très significatif dans un système naturel, qui constitue le modèle d'évaluation de l'effet protecteur de MSP-1 le plus représentatif pour l'homme.

L'effet protecteur obtenu pourrait être d'autant meilleur que la forme p19 est dépourvue de la région hypervariable de la partie N-terminale du p42, dont l'effet peut être délétère dans des situations naturelles dans lesquelles le sujet vacciné est confronté à un polymorphisme important. Aussi la p19 semble posséder des épitopes spécifiques qui ne sont pas présents dans la p42.

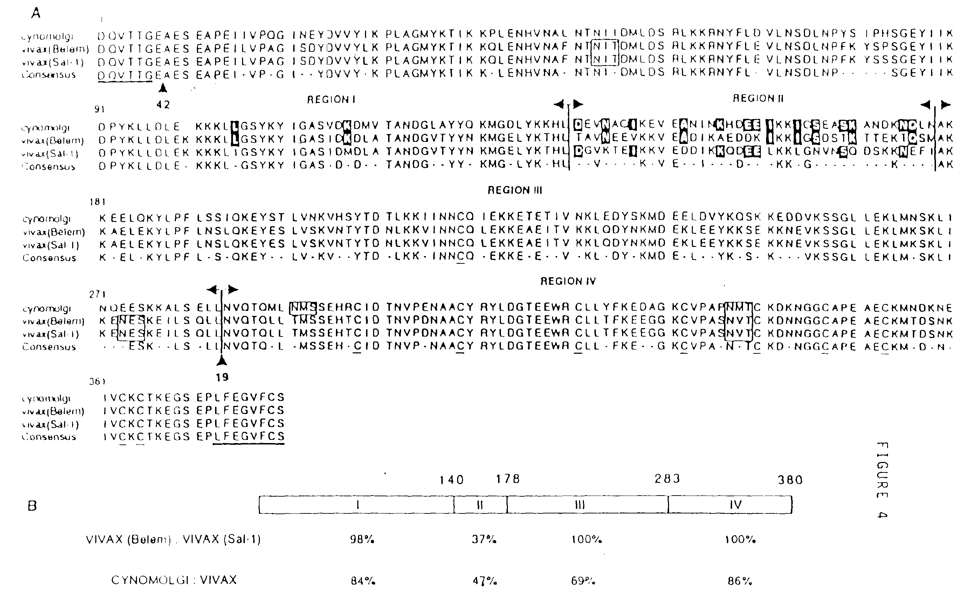

Le fragment C-terminal de 19 kDa dont !a séquence est présente dans le principe actif du vaccin peut être limité à la séquence de la p19 elle-même, en l'absence de toute séquence polypeptidique normalement en amont de la séquence de p19 dans la protéine MSP-1 correspondante. Il va néanmoins de soi que la séquence polypeptidique constitutive essentielle du principe actif peut encore comporter une séquence polypeptidique du côté C-terminal appartenant au fragment N-terminal de 33 kDa (p33) encore associée à la p19 dans la p42 correspondante, avant le clivage naturel de cette dernière, et cela chaque fois que la présence de ce fragment n'est pas de nature à modifier les propriétés immunologiques du principe actif du vaccin. Comme cela sera vu plus loin, notamment à propos de la description des exemples, les séquences C-terminales de la p33 de souches diverses d'une même espèce de *Plasmodium* (voir la partie C-terminale des séquences peptidiques de la « région III » de la figure 4), présentent aussi encore un degré d'homologie ou de conservation substantielle de la séquence, par exemple de l'ordre de 80% au moins, dans différentes variétés de *Plasmodiums* infectieux pour l'homme, de sorte qu'elles ne sont pas de nature à modifier fondamentalement les propriétés vaccinantes du principe actif (dont la séquence correspond à la région IV) de la figure 4, en particulier dans l'hypothèse qui découle de cette figure le site de clivage présumé entre la p19 et la région III de la p33 se situe entre les résidus leucine et asparagine dans une région particulièrement bien conservée (LNVQTQ).

Normalement la séquence polypeptidique C-terminale de la p33, lorsqu'elle est présente, comprend moins de 50 résidus d'acides aminés, voire même moins de 35, ou même moins de 10 résidus d'acides aminés.

A l'inverse, la séquence polypeptidique constitutive essentielle du principe actif de vaccin peut ne pas comporter la totalité de la séquence codant pour la p19, naturellement sous réserve que cette dernière conserve la capacité d'induire des anticorps protecteurs contre le parasite. En particulier la susdite « partie de fragment a un poids moléculaire de 10 à 25 kDa, notamment de 10 à 15 kDa. De préférence, cette partie de fragment polypeptidique contient au moins l'une des deux régions EGF (abréviation de l'expression anglaise « Epidermal Growth Factor »).

Il est clair que l'homme du métier est à même de faire la différence entre les fragments actifs et ceux qui cesseraient de l'être, notamment de façon expérimentale en produisant des vecteurs modifiés contenant des insérats issus de la p19 de longueurs différentes, respectivement isolés à partir des fragments obtenus à partir de la séquence codante pour la p19, par réaction avec des enzymes de restriction appropriés, ou encore par des enzymes exonucléolytiques qui auraient été maintenus au contact du fragment codant pour la p19 pendant des temps variables ; la capacité des produits d'expression de ces insérats dans des cellules eucaryotes correspondantes, notamment des cellules d'insectes, transformées par les vecteurs modifiés correspondants, à exercer un effet protecteur, pouvant alors être testée, notamment dans les conditions expérimentales qui seront décrites plus loin, à propos des exemples. En particulier, les produits d'expression de ces insérats doivent être aptes à inhiber une parasitémie induite *in vivo* par le parasite correspondant entier.

De même, l'invention inclut toutes compositions vaccinantes dans lesquelles la séquence polypeptidique constitutive essentielle du principe actif serait constitué d'un peptide capable d'induire une réponse immunologique de type cellulaire et/ou humorale équivalente à celle produite par la p19 ou au fragment tel qu'il vient d'être défini, dès lors que l'addition, la délétion ou la substitution dans sa séquence de certains acides aminés par d'autres n'entraîneraient pas une modification importante de la capacité du peptide ainsi modifié -ci-après appelé « peptide immunologiquement équivalent »- à aussi inhiber la susdite parasitémie.

Le fragment de la p19 peut naturellement aussi être associé que ce soit du côté N-terminal ou du côté C-Terminal ou par l'intermédiaire d'une liaison peptidique à un autre fragment de protéine plasmodiale ayant un potentiel vaccinant comme par exemple une protéine (« Duffy Binding Protein » de P. vivax (29) ou EBA-175 de P. falciparum (30) et (31) dont une région est spécifiquement riche en cystéine), sous réserve que ne soit pas altérée, au contraire amplifiée, sa capacité d'inhiber une parasitémie normalement introduite in vivo par le parasite correspondant.

Le fragment codant pour la p19 ou une partie de celle-ci, peut contenir également, en amont de l'extrémité N-terminale de p19, une séquence peptidique encore différente, par exemple à un fragment C-terminal du peptide signal utilisé, tel que celui de la protéine MSP-1. Cette séquence comprend de préférence moins de 50 acides aminés, par exemple de 10 à 40 acides aminés.

Ces observations s'étendent de la même façon à des p19 issues d'autres *Plasmodium,* en particulier *P.falciparum*, l'espèce dominante des parasites, responsable d'une des formes les plus graves de paludisme.

Mais les techniques rappelées ci-dessus pour la production dans un système de baculovirus d'une p19 recombinante issue de *P.vivax* ou *P.cynomolgi* sont difficilement transposables telles quelles à la production d'une p19 recombinante de *P. falciparum* avec un rendement satisfaisant, ne fût-ce que pour obtenir des quantités appréciables autorisant la réalisation d'essais d'immunoprotection

L'invention fournit également un procédé remédiant en grande partie à cette difficulté Il devient aussi possible d'obtenir des rendements beaucoup plus importants en p19 de *P.falciparum* -et d'autres *Plasmodiums* lorsque l'on rencontre des difficultés semblables- en mettant en oeuvre une séquence nucléotidique synthétique de substitution à la séquence nucléotidique naturelle codant pour la p19 de *Plasmodium falciparum* dans un vecteur d'expression d'un système baculovirus, cette séquence nucléotidique synthétique codant pour la même p19, mais étant caractérisée par une proportion de nucléotides G et C plus élevée que dans la séquence nucléotidique naturelle.

En d'autres termes, l'invention découle de la découverte que l'expression dans un système baculovirus d'une séquence nucléotidique codant pour une p19, était apparemment liée à une compatibilité améliorée des codons successifs de la séquence nucléotidique à exprimer avec la « machinerie cellulaire » des cellules hôtes transformables par des baculovirus, à l'instar de ce qui est observé pour les séquences nucléotidiques naturelles normalement contenues dans ces baculovirus et exprimées dans les cellules hôtes infectées ; d'où la mauvaise expression, sinon parfois l'absence totale d'expression d'une séquence nucléotidique native de *P.falciparum ;* d'où également une explication possible à l'expression plus efficace observée de la p19 de *P.vivax* dans un système baculovirus par Longacre et al. (14) et, comme les inventeurs l'ont aussi constaté, de la séquence de *P.cynomolgi à* partir des séquences nucléotidiques p19 natives correspondantes, en raison de leurs teneurs relatives en nucléotides G et C beaucoup plus élevées que celles des séquences nucléotidiques natives codant pour les p19 de *P.falciparum.*

L'invention concerne donc aussi, plus généralement, un vecteur modifié du type baculovirus recombinant contenant sous le contrôle d'un promoteur contenu dans ce vecteur et susceptible d'être reconnu par des cellules transfectables par ce vecteur, une première séquence nucléotidique codant pour un peptide signal exploitable par un système baculovirus, caractérisé par une deuxième séquence nucléotidique en aval de la première, également sous le contrôle de ce promoteur et code pour la séquence peptidique :
- soit d'un fragment peptidique C-terminal de 19 kilodaltons (p19) de la protéine 1 de surface (protéine MSP-1) de la forme mérozoïte d'un parasite du type *Plasmodium* autre que *Plasmodium vivax* et infectieux pour l'Homme ce fragment C-terminal restant normalement ancré à la surface du parasite au terme de sa pénétration dans des érythrocytes humains, à l'occasion d'un cycle infectieux ;
- soit d'une partie de ce fragment peptidique dès lors que le produit d'expression de la deuxième séquence dans un système baculovirus est apte aussi à induire une réponse immune capable d'inhiber une parasitémie in vivo due au parasite correspondant ;
- soit d'un peptide immunologiquement équivalent dérivé du susdit fragment peptidique C-terminal (p19) ou de la susdite partie de fragment peptidique par addition, délétion ou substitution d'acides aminés n'entraînant pas une modification importante de la capacité de ce peptide immunologiquement équivalent à induire une réponse immunologique de type cellulaire et/ou humorale semblable à celle produite par ce fragment peptidique p19 ou à la susdite partie de ce fragment, et
   ladite séquence nucléotidique ayant le cas échéant une teneur en nucléotides G et C comprise entre 40 et 60%, de préférence d'au moins 50% de la totalité des nucléotides dont elle est constituée. Cette séquence peut être obtenue par construction d'un gène synthétique dans lequel les codons naturels ont été changés par des codons riches en G/C sans que leur traduction soit modifiée (maintien de la séquence peptidique).

En l'occurrence ladite séquence nucléotidique, fournie par un ADN synthétique, peut présenter au moins 10% de codons modifiés par rapport à la séquence du gène ou du cDNA naturel tout en conservant les caractéristiques de la séquence naturelle traduite, c'est-à-dire le maintien de la séquence en amino-acides.

Cela étant, on n'exclut pas que cette teneur en nucléotides G et C pourrait être davantage accrue, dès lors que les modifications qui en résulteraient quant à la séquence en acides aminés du peptide recombinant -ou peptide immunologiquement équivalent-produit n'entraîneraient pas une perte des propriétés immunologiques, voire protectrices, des protéines recombinantes formées, notamment dans les essais qui seront illustrés plus loin.

Ces observations s'appliquent naturellement à d'autres *Plasmodium* infectieux pour l'homme, en particulier dès lors que des séquences nucléotidiques natives codant pour les p19 correspondantes, auraient des teneurs en nucléotides T et A difficilement compatibles avec une expression efficace dans un système baculovirus.

La séquence codant pour le signal utilisé peut être celle normalement associée à la séquence native du *Plasmodium* concerné. Mais elle peut également être issue d'un autre *Plasmodium,* par exemple *P.vivax* ou *P.cynomolgi* ou d'un autre organisme s'il est susceptible d'être reconnu en tant que signal dans un système baculovirus.

La séquence codant pour la p19 ou un fragment de celle-ci au sein du vecteur considéré est, le cas échéant, dépourvue de la séquence d'ancrage de la protéine native au parasite dont elle est issue, cas dans lequel la protéine exprimée est en général excrétée dans le milieu de culture (forme soluble). Il est d'ailleurs remarquable à cet égard que dans les conditions de l'invention, les formes solubles et ancrées de protéines recombinantes produites, en particulier lorsqu'elles sont issues de *P.falciparum* ou *P.cynomolgi* ou *P.vivax,* ont tendance à former des oligomères, cette propriété pouvant être à l'origine des immunogénicités accrues des protéines recombinantes formées.

L'invention concerne tout autant les vecteurs dans lesquels la séquence codante contient la séquence d'extrémité 3' terminale codant pour la séquence d'extrémité C'-terminale hydrophobe de la p19 et qui est normalement impliquée dans l'induction de l'ancrage de la protéine native à la membrane cellulaire de l'hôte dans lequel elle est exprimée. Cette région d'extrémité 3'-terminale peut d'ailleurs être hétérologue vis-à-vis de la séquence codant pour la partie soluble de p19, par exemple correspondre à la séquence 3'-terminale issue de *P.vivax* ou d'un autre organisme dès lors qu'elle code pour une séquence d'ancrage de l'ensemble de la protéine recombinante produite à la membrane de l'hôte cellulaire du système baculovirus mis en oeuvre. A titre d'exemple de telles séquences d'ancrage on cite la GPI de l'antigène CD59 exprimable dans des cellules d'insectes du type *Spodoptera frugiperda* (32) ou la GPI d'une protéine humaine CD14 (33).

L'invention concerne naturellement aussi les protéines recombinantes, ces protéines comportant des épitopes conformationnels reconnus par des sérums humains formés contre le *Plasmodium* correspondant

D une façon générale, l'invention concerne également toute protéine recombinante du type indiqué ci-dessus, dès lors qu'elle comporte des épitopes conformationnels tels que produits dans le système baculovirus, notamment ceux qui se trouvent être instables en milieu réducteur.

L'invention concerne naturellement lesdites protéines recombinantes, qu'elles soient sous la forme dite soluble ou sous la forme pourvue d'une région d'ancrage, en particulier aux hôtes cellulaires mis en oeuvre dans le système baculovirus.

Entrent également dans le champ de l'invention, les oligomères produits spontanément dans les systèmes baculovirus utilisés ou produits a posteriori, en ayant recours à des techniques classiques d'oligomérisation de protéines. La technique couramment utilisée met en oeuvre le glutaraldéhyde. Mais tout système classique de pontage entre des fonctions respectivement amine et carboxyle, telles qu'on les retrouvent dans les protéines pourra également être utilisé. A titre d'exemples, on peut mettre en oeuvre l'une quelconque des techniques décrites dans la demande de brevet européen 0602079.

Par « oligomère » on entend une molécule contenant de 2 à 50 unités monomère, chacune de ces unités monomère contenant la p19 ou un fragment de celle-ci, tel que défini ci-dessus, capable de former un agrégat L'invention porte également sur tout produit de conjugaison entre une p19 ou fragment de p19 tel que défini ci-dessus, d'une part, et une molécule porteuse -par exemple une polylysine-alanine- utilisable pour la production des vaccins, d'autre part, par l'intermédiaire de liaisons covalentes ou non. Les compositions vaccinantes les mettant en oeuvre font également partie de l'invention.

L'invention concerne également les compositions de vaccins mettant en oeuvre ces protéines recombinantes oligomères ou conjugués, y inclus d'ailleurs les protéines issues de *Plasmodium vivax,* ces observations s'étendant également aux oligomères de ces protéines recombinantes.

Font également partie de l'invention les compositions dans lesquelles les protéines recombinantes susmentionnées sont associées à un adjuvant, par exemple un alun. Les protéines recombinantes comportant l'extrême région C-terminale permettant leur ancrage à la membrane des cellules dans lesquelles elles sont produites sont avantageusement utilisées en combinaison avec des lipides aptes à former des liposomes et appropriés à la production de vaccins. Sans qu'il y ait eu lieu de s'y limiter, on peut avoir recours aux lipides décrits à cet effet par exemple dans l'ouvrage intitulé « Les liposomes aspects technologique, biologique et pharmacologique » de J. Delattre et al., édition INSERM, 1993.

La présence de la région d'ancrage dans la protéine recombinante, qu'il s'agisse d'une région d'ancrage homologue ou hétérologue vis-à-vis de la partie vaccinante proprement dite, est de nature à favoriser la production d'anticorps cytophiles, notamment du type IgG₂ₐ et IgG_{2b} chez la souris qui pourraient avoir une activité protectrice particulièrement élevée, au point que l'on pourrait se dispenser d'associer les principes actifs de vaccins ainsi constitués avec des adjuvants autres que les lipides utilisés pour la constitution des formes liposomiques. Il s'agirait là d'un avantage important, puisque les liposomes peuvent être lyophilisés dans des conditions permettant leur stockage et leur transport, sans que des chaînes de froid ne soient alors indispensables.

D'autres caractéristiques de l'invention apparaîtront encore au cours de la description qui suit d'exemples de protéines recombinantes entrant dans le cadre de l'invention et des conditions dans lesquelles elles peuvent être produites, sans que ces exemples soient de nature à limiter la portée de l'invention

### Description de la construction PfMSP1ₚ₁₉S (soluble) (p19 soluble issue de P.falciparum)

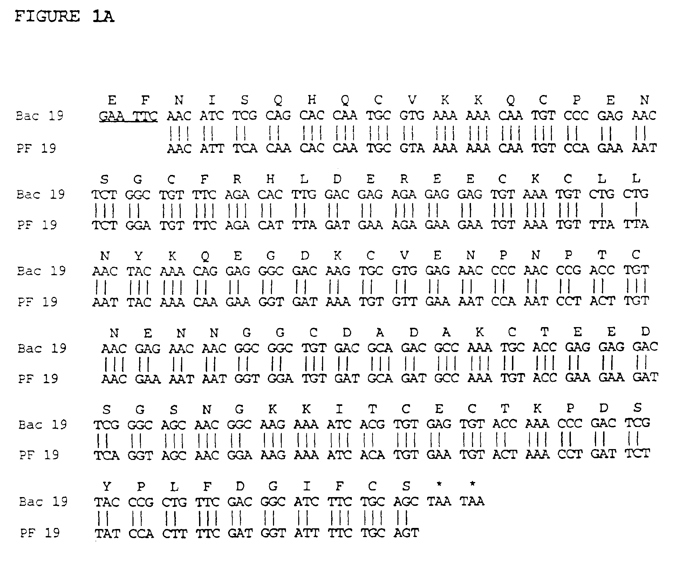

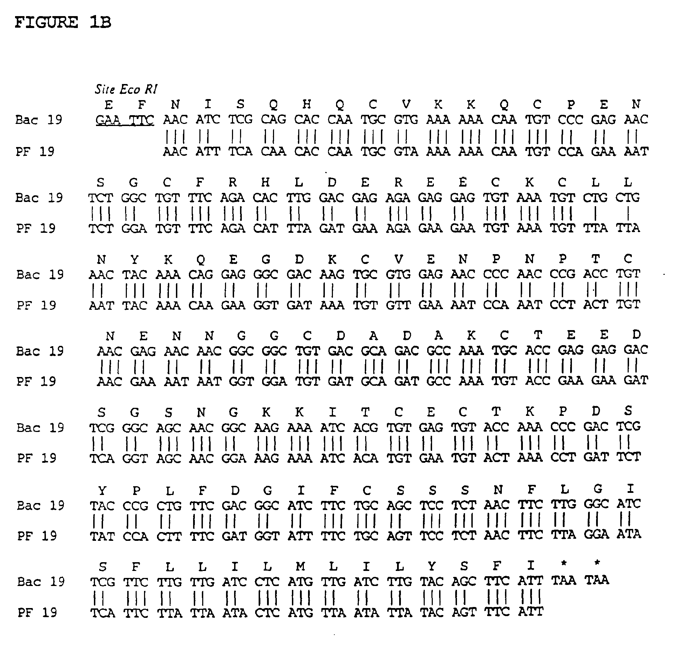

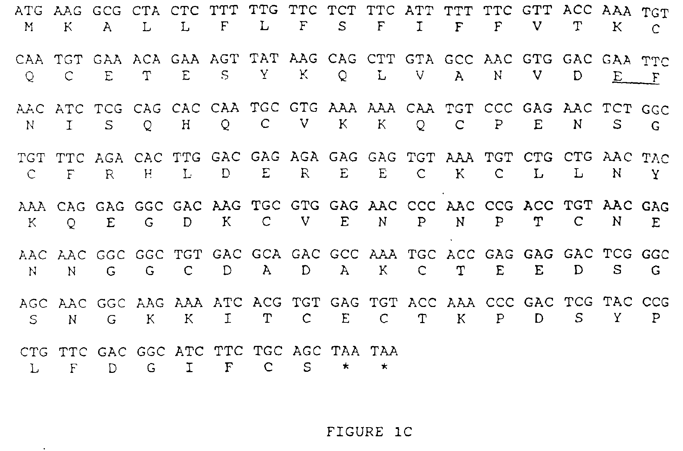

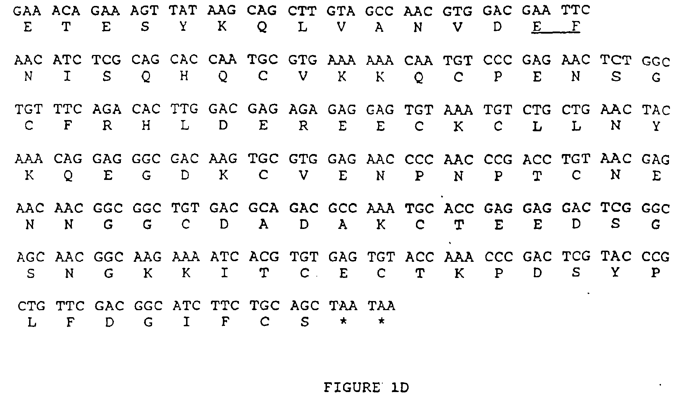

La construction recombinante PfMSP1ₚ₁₉S contient de l'ADN correspondant aux 8 paires de base de la séquence leader et les 32 premiers acides aminés de MSP1 de *Plasmodium vivax* de Met₁ à Asp₃₂ (isolat Belem ; Del Portillo et al 1991 P.N.A.S. 88, 4030.) suivis par un GluPhe, dû au site EcoR1 faisant la liaison des deux fragments. Le tout est suivi par le gène synthétique, décrit dans Figure 1, codant le *Plasmodium falciparum* MSP1ₚ₁₉ de Asn₁₆₁₃ à Ser₁₇₀₅ (isolat Uganda-Palo Alto ; Chang et al. 1988. Exp. Parasitol. 67,1). La construction est terminée par deux codons stop de TAA Cette construction donne lieu à une protéine recombinante qui est sécrétée dans le surnageant de culture des cellules infectées.

De la même façon et à des fins de comparaisons, on a produit une construction recombinante dans des conditions semblables à celles utilisées pour la production de la p19 ci-dessus, mais en travaillant avec une séquence codante consistant en une copie directe de l'ADN correspondant de la souche *P.falciparum* (FUP) décrite par Chang et al , Exp Parasit. 67,1: 1989. La copie de ce gène naturel (s'étendant de l'asparagine 1613 à la sérine 1705) a été formée par PCR à partir du gène natif

On a représenté dans la Figure 1A les séquences à la fois du gène synthétique (Bac19) et du « gène natif » (PF19)

On remarque que 57 codons sur les 93 codons de la séquence codante native pour la p19 issue de *P.falciparum* ont été modifiés (pour ce qui est du troisième nucléotide dans 55 d'entre eux et du premier et troisième nucléotides dans les deux codons restant). Des codons nouveaux ont été ajoutés à l'extrémité 5' pour introduire le peptide signal dans les conditions qui ont été indiquées ci-dessus et pour introduire un site EcoRI pour le clonage, d'une part, et de même ont été ajoutés deux codons stop non présents dans la p19 de *P.falciparum* aux fins d'obtenir des signaux de terminaison de l'expression. Les lettres individualisées placées respectivement au dessus des codons successifs correspondent aux acides aminés respectifs successifs. Les astérisques (*) se rapportent aux codons stop. Les lignes verticales soulignent les nucléotides qui sont les mêmes dans les deux séquences.

### Description de la construction PfMSP1ₚ₁₉A (ancré GPI) (p19 ancrée de P.falciparum)

La construction PfMSP1ₚ₁₉A a des caractéristiques de la précédente sauf que la séquence synthétique (Figure 1 B) code pour le MSP1ₚ₁₉ de *Plasmodium falciparum* (isolat Uganda-Palo alto) de Asn₁₆₁₃ à Ile₁₇₂₆ suivie par deux codons stop de TAA. Cette construction donne lieu à une protéine recombinante qui est ancrée dans la membrane plasmique des cellules infectées par une structure du type glycosyl phosphatidyl inositol (GPI).

La **figure 1C** est représentative de la séquence de la protéine recombinante PfMSP1_{P19}S avant coupure de la séquence signal.

La **figure 1D** est représentative de la séquence de la protéine recombinante PfMSP1ₚ₁₉S après coupure de la séquence signal.
Les acides aminés soulignés dans les figures 1C et 1D proviennent du site EcoR1 utilisé pour joindre les séquences nucléotidiques dérivées de la partie N-terminale de MSP1 de *P.vivax* (avec séquence signal) et de MSP1ₚ₁₉ de *P.falciparum.*

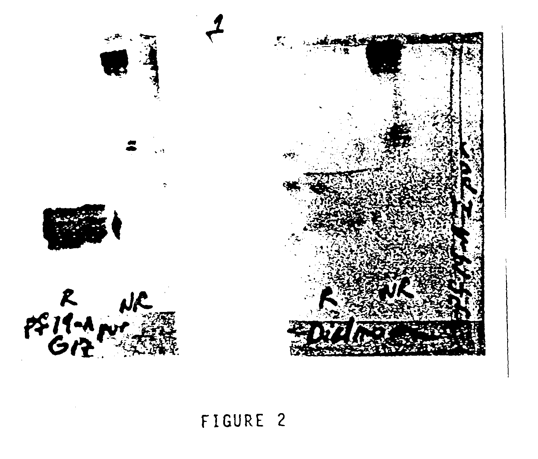

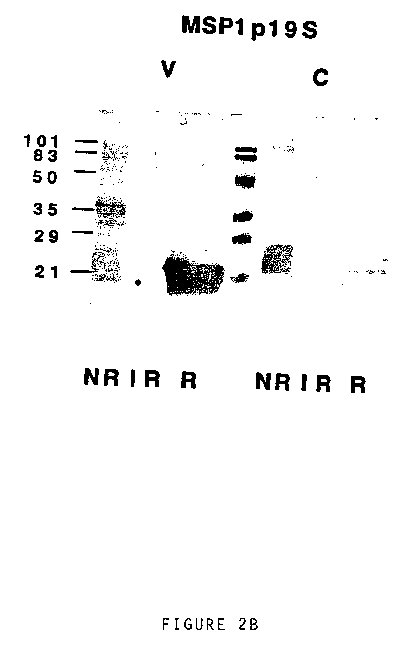

**Figure 2** - L'antigène recombinant PfMSP1ₚ₁₉ soluble purifié par immunoaffinité a été analysé par immunoblot après SDS-PAGE en présence (réduit) ou absence (non réduit) de B-mercaptoéthanol. Les échantillons sont chargées sur gel après chauffage à 95°C en présence de 2% SDS Dans ces conditions seulement des liaisons du type covalent (ponts disulfure) peuvent résister à la désagrégation. Le blot de gauche a été révélé avec un anticorps monoclonal qui réagit avec un épitope linéaire de la p19 naturelle. Le blot de droite a été révélé avec un mélange de 13 antisera humains provenant des sujets avec une immunité acquise au paludisme dû à *Plasmodium falciparum.* Ces résultats montrent que la molécule recombinante de baculovirus reproduit bien les épitopes conformationnels en forme de polymère qui sont reconnus en majorité par l'antiserum humain.

**Figure 2B** Analyse par immunoblot avec antisérum humain du MSP-1 p19 purifié de *P. vivax* et *P. cynomolgi* recombinant dans des conditions non-réduites (NR), réduite seulement dans le milieu de chargement (R) et réduite irréversiblement (IR):
Ce travail est basé sur l'idée que le système d'expression baculovirus reproduit correctement et en grande proportion les épitopes conformationnels présents *in vivo* sur la partie C-terminale de MSP-1. Le meilleur moyen de mesurer cette propriété (et peut être le seul moyen possible en absence des protéines natives purifiées correspondant au p19) est d'étudier la réactivité des protéines recombinantes avec l'antisérum des individus exposés au paludisme, ceci étant un reflet des protéines natives telles qu'elles sont "vues" par le système immunitaire humain
Donc, les antigènes recombinants PvMSP-1 p19 et Pc MSP-1 p19 solubles purifié par immunoaffinité ont été analysé par immunoblot après SDS-PAGE (15%) en présence (réduit) ou absence (non-réduit) de DTT. Les échantillons sont chargées sur gel après chauffage à 95°C en présence de 2% SDS. La réduction irréversible était faite comme suit: la protéine est resuspendue en 0.2 M Tris-HCl, pH 8.4, 100 mM DTT, 1.0% SDS et chauffée 30 minutes à 70°C. Après dilution avec l'eau, de l'acrylamide est ajouté à une concentration finale de 2 M et la mixture est incubée sous azote sans lumière 1 heure à 37°C. L'immunoblot a été révélé avec un mélange de 25 antisera humains provenant des sujets avec une immunité acquise au paludisme dû à *Plasmodium vivax.* V et C désigne respectivement les protéines dérivées de MSP-1 de *P*. *vivax* et *P*. *cynomolgi.* Il est remarquable que les protéines recombinantes réduites de façon irréversible ne montrent aucune réactivité avec l'antisérum humain tandis que les protéines réduites de façon non-irréversible ou non-réduites montrent une bonne réactivité. (Le Pv MSP-1 p19 non-réduit est un peu faible parce que dans son état glycosylé il ne se lie pas très bien au papier nitrocellulose.) Ces résultats montrent que la reconnaissance des molécules MSP-1 p19 de baculovirus par l'antisérum humain est en grande partie, sinon complètement dépendant des épitopes conformationnels sensibles à la réduction qui sont reproduits dans ce système.

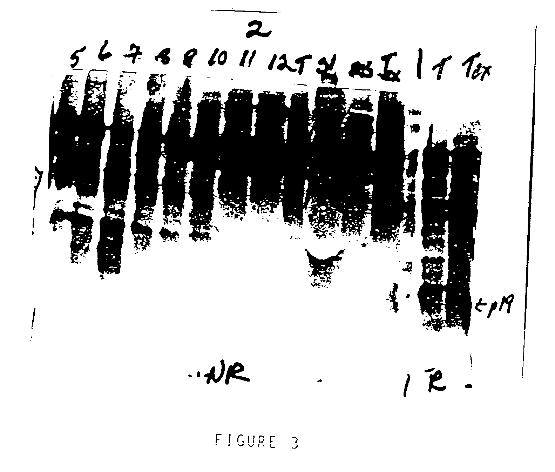

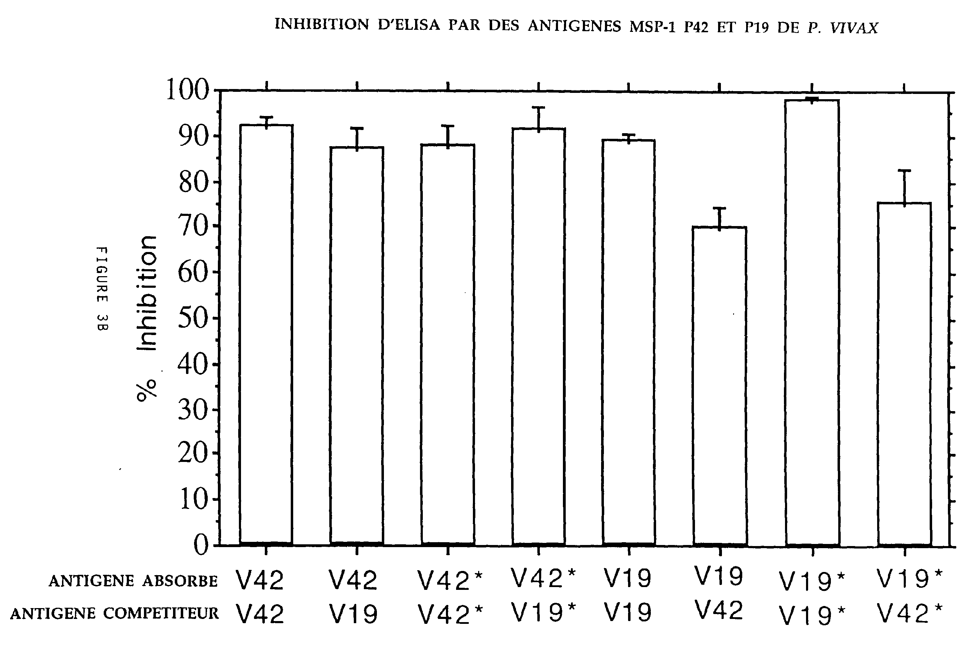

**Figure 3** - L'antigène recombinant PvMSP1_{P42} soluble (Longacre et al. 1994, op.cit.) a été incubé pendant 5 heures à 37° en présence des fractions de protéines dérivées des mérozoïtes de *P.falciparum* et séparées par isoélectrofocalisation. Par la suite les échantillons ont été analysés par immunoblot en présence (réduit) ou absence (non réduit) de B-mercaptoéthanol. Les fractions 5 à 12 d'isoélectrofocalisation, ainsi que deux extraits totaux de mérozoïtes faits en présence (Tex) ou absence (T) de détergent ont été analysés. L'immunoblot a été révélé avec des anticorps monoclonaux spécifiques pour le MSP1ₚ₄₂ et ₚ₁₉ de *P.vivax.* Les résultats suggèrent qu'il y a une activité protéolytique dans les mérozoïtes de *P.falciparum* qui peut être extraite en détergent. La digestion du p42 dans certaines fractions semble provoquer une polymérisation des produits de digestion (p19) ; cette polymérisation est probablement liée à la formation de ponts disulfure puisqu'en présence de B-mercaptoéthanol, les formes de haut poids moléculaire disparaissent en faveur d'une molécule d'environ 19 kDa (Tex-R). La polymérisation du p19 observée dans ces expériences pourrait donc être une propriété intrinsèque de cette molécule *in vivo.*

**Figure 3B**: La contribution différentielle des antigènes p42 et p19 à la réponse humaine anti-MSP-1 de *P. vivax.*
La reconnaissance des antigènes MSP-1 p42 et p19 de *P. vivax* par l'antisérum des individus ayant une immunité acquise à P. *vivax* a été comparée par la technique d'inhibition d'ELISA comme suit: un mélange de 25 antisérums humains provenant des sujets avec une immunité acquise au paludisme dû à *P. vivax* a été dilué au 1:5000 et incubé 4 heures à température ambiante soit seul, soit en présence d'une solution de 1 mM p42 ou p19 recombinant purifié de *P*. *vivax.* Cette mixture a été transférée dans un puits de microtitre auparavant revêtu pendant 18 heures à 4°C par 500 ng ml⁻¹ p42 ou p19 recombinant purifié absorbé, et incubée 30 minutes à température ambiante. Après lavage avec PBS contenant 0 1 % Tween 20, un anti-souris IgG de chèvre conjugué avec peroxydase a été ajouté et la mixture a été incubée 1 heure à 37°C et l'activité enzymatique a été révélée par lecture de la densité optique à 492 nm La pourcentage d'inhibition a été calculée basé sur des valeurs de 100%, de réactivité d'antisérum avec l'antigène revêtu en plaque de microtitre en absence d'un antigène compétiteur Les données statistiques ont été calculées en utilisant le programme Statview. Chaque barre représente l'inhibition moyenne en pourcentage d'une paire d'antigènes compétiteur / absorbé basée sur 4 à 12 déterminations et les lignes verticales correspondent à un intervalle de 95% de confiance. Les étoiles (*) désignent des antigènes produits en présence de tunicamycine, donc sans N-glycosylation. Les paramètres importants de ces mesures sont la dilution d'antisérum à 1:5000 qui est dans la région sensible des courbes d'ELISA et les concentrations d'antigènes compétiteurs à 1 mM qui incluent la compétition par les épitopes de basse affinité. Donc, les données reflètent la similitude maximum entre les deux antigènes comparés. Les résultats montrent que la plupart, si ce n'est pas tous les épitopes du p42 reconnus par l'antisérum humain sont présents sur le p19 puisqu'en présence de ce dernier, la réactivité de l'antisérum humain contre le p42 est inhibée autant que par l'antigène p42 lui même. Mais, au contraire, environ 20% des épitopes du p19 reconnus par l'antisérum humain ne le sont pas ou ne sont pas accessibles sur le p42, puisque la réactivité de l'antisérum humain contre le p19 est bien moins inhibée par le p42 que par le p19 lui même. Des tels épitopes spécifiques du p19 peuvent être constitué ou révélés seulement après la coupure du p42 en p19 et p33. Ces résultats ne sont pas affectés par la glycosylation montrant que l'effet est bien dû à une différence entre les composants peptidiques du p19 et p42 et pas à une différence de glycosylation. Ces résultats souligne le fait que le p19 a une identité immunologique distincte du p42.

### Description de la construction PcMSP1ₚ₁₉S (soluble) (p19 soluble de P.cynomolgi)

L'ADN utilisé pour la construction susdite a été obtenu à partir d'un clone de la souche de *Plasmodium cynomolgi ceylonesis* (22-23). Cette souche a été maintenue par des passages successifs dans son hôte naturel (*Macaca sinica*) et des transmissions cycliques par l'intermédiaire de moustiques (27).

Des parasites sanguins ont été obtenus à partir des singes infectés au stade schizonte mature quand les parasitémies ont atteint un niveau de 5% Ils ont alors été purifiés selon la méthodes décrites dans (25) L'ADN a ensuite été extrait comme décrit dans (26)

Un fragment de 1200 paires de base a ensuite été produit en ayant recours à la réaction PCR mettant en oeuvre les oligonucléotides soulignés dans la Figure 4 et issus de *P.vivax.* L'oligonucléotide 5' comprenait un site de restriction EcoRI et l'oligonucléotide 3' deux codons synthétiques stop TAA suivis d'un site de restriction BglII. Ce fragment a été introduit par ligation et par l'intermédiaire de ces sites EcoRI et BglII dans le plasmide pVLSV₂₀₀. contenant déjà la séquence signal de la protéine MSP-1 de *P.vivax* (19). Le nouveau plasmide (pVLSV₂₀₀C₄₂) a été utilisé pour l'analyse de séquences d'ADN

Les séquences de *P.cynomolgi* et des séquences correspondantes de *P.vivax* ont été mises en alignement. Les flèches noires désignent les sites de clivage primaire et secondaire présumés. Ils ont été déterminés par analogie avec les sites connus dans *P.falciparum* (27, 28). Des lignes verticales et des flèches horizontales localisent les limites des quatre régions qui ont été étudiées. La région 4 correspond à la séquence codant pour la p19 de *P.cynomolgi.* Des sites de glycosylation sont encadrés et les cystéines conservées sont soulignées Dans la partie inférieure de la **Figure 4** Sont indiqués leS pourcentages identité entre les deux isolats de *P.vivax* et *P.cynomolgi.*

La construction recombinante PcMSP1ₚ₁₉S contient de l'ADN correspondant aux 8 paires de bases de la séquence « leader » et les 32 premiers acides aminés de MSP1 de *Plasmodium vivax* de Met₁ à Asp₃₂ (isolat Belem ; Del Portillo et al. 1991 P.N.A.S. 88, 4030.) suivis par un GluPhe, dû au site EcoR1 faisant la liaison des deux fragments. Le tout est suivi par la séquence codant pour le MSP1ₚ₁₉ de *Plasmodium cynomolgi* (souche Ceylon) de Lys₂₇₆ à Ser₃₈₀. La construction est terminée par deux codons stop de TAA. Cette construction donne lieu à une protéine recombinante qui est sécrétée dans le surnageant de culture des cellules infectées

### Purification de la protéine recombinante PfMSP1p19 par chromatographie d'immunoaffinité avec un anticorps monoclonal reconnaissant spécifiquement la p19 de Plasmodium falciparum.

La résine de chromatographie a été préparée en liant 70 mg d'un anticorps monoclonal (obtenu à partir d'un hybridome G17.12 déposé à la CNCM (Paris, France) le 14 février 1997 sous le n°I-1846 ; cet hybridome G17.12 a été construit à partir du myélome X63 Ag8 653 produisant des IgG 2a/k reconnaissant la p19 de *P.falciparum*) à 3g de CNBr-Sepharose 4B activé (Pharmacia) par des méthodes standards détaillées dans le mode d'emploi fourni par Pharmacia. Les surnageants de culture contenant le PfMSP1p19 soluble ont été incubés en batch avec la résine de chromatographie pendant 16 heures à 4°C. La colonne a été lavée une fois avec 20 volumes de 0.05% NP40, 0.5M NaCl, PBS ; une fois avec 5 volumes de PBS et une fois avec 2 volumes de 10 mM phosphate de sodium, pH 6.8. L'élution a été effectuée avec 30 ml de 0.2 M glycine, pH 2.2. L'éluat a été neutralisé avec 1 M phosphate de sodium, pH 7.7 puis concentré par ultrafiltration et dialysé contre du PBS. Pour la purification du PfMSP1p19 ancré, toutes les solutions de lavage et élution contenaient en supplément 0.1% 3-(Dimethyl-dodecylammonio)-propane sulfonate (Fluka).

### Essai de vaccination de MSP1 recombinant de Plasmodium vivax (p42 et p19) chez le singe écureuil Saimiri sciureus.

Cet essai de vaccination a été fait chez des *Saimiri sciureus boliviensis* mâles de 2 à 3 ans, non splénectomisés. Trois singes ont été injectés 3 fois par voie intramusculaire à 3 semaines d'intervalle avec un mélange d'environ 50 à 100 µg chacun, de PvMSP1ₚ₄₂ et ₚ₁₉ soluble recombinant (19), purifié par immunoaffinité. L'adjuvant de Freund complet et incomplet était utilisé comme suit : 1^{ère} injection: 1:1 FCA/FIA ; 2^{ème} injection 1:4 FCA/FIA ; 3^{ème} injection : FIA. Ces compositions d'adjuvant étaient mélangées par la suite 1:1 avec l'antigène en PBS. Les cinq singes contrôles recevaient l'antigène glutathione-S-transterase (GST) produit dans *E.coli* selon le même protocole. L'infection d'épreuve était effectuée en injectant 2.10⁶ hématies infectées avec une souche adaptée de *Plasmodium vivax* (Belem) 2.5 semaines après la dernière injection. La protection a été évaluée en déterminant les parasitémies journalières chez tous les animaux par examen des frottis colorés avec giemsa.

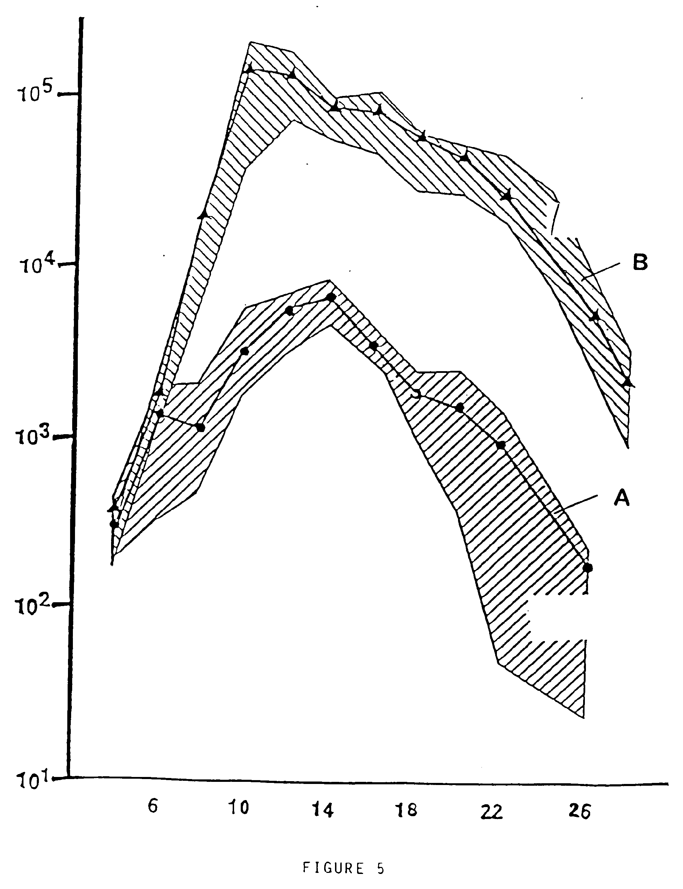

Les courbes de la Figure 5 sont représentatives de la variation de la parasitémie mesurée en nombre d'hématies parasitées par microlitre de sang (sur l'axe des ordonnées à l'échelle logarithmique) en fonction du temps écoulé après l'infection (en jours). La courbe A correspond aux valeurs moyennes observées chez les trois singes vaccinés, la courbe B ; les valeurs moyennes chez cinq singes témoins.

De l'examen de la figure découle une très forte réduction de la parasitémie sous l'effet de la vaccination

### Essai de vaccination de MSP1 recombinant de Plasmodium cynomolgi (p42 et p19) chez le singe toque, Macaca sinica.

Quinze singes capturés ont été utilisés comme suit : (1) 3 animaux injectés avec 100 µg PcMSP1ₚ₄₂ soluble ; 3 animaux injectés avec 35 µg (1^{ere} injection) ou 50 µg (2^{ème} et 3^{eme} injections) PcMSP1ₚ₁₉ soluble ; **(3)** 3 animaux injectés avec un mélange de PcMSP1ₚ₄₂ et ₚ₁₉; (4) 3 animaux injectés avec l'adjuvant plus PBS , (5) 3 animaux non injectés. L'adjuvant de Freund complet et incomplet a été utilisé selon le protocole décrit ci-dessus Les injections on été faites par voie intramusculaire à 4 semaines d'intervalle L'infection d'épreuve était faite en injectant 2.10⁵ hématies infectées avec *Plasmodium cynomolgi* 4 semaines après la dernière injection La protection a été évaluée en déterminant les parasitémies journalières chez tous les animaux en examinant les parasitémies avec giemsa. Les parasitémies ont été classées comme négatives uniquement après comptage de 400 champs de frottis. Les parasitémies sont exprimées en pourcentage d'hématies parasitées.

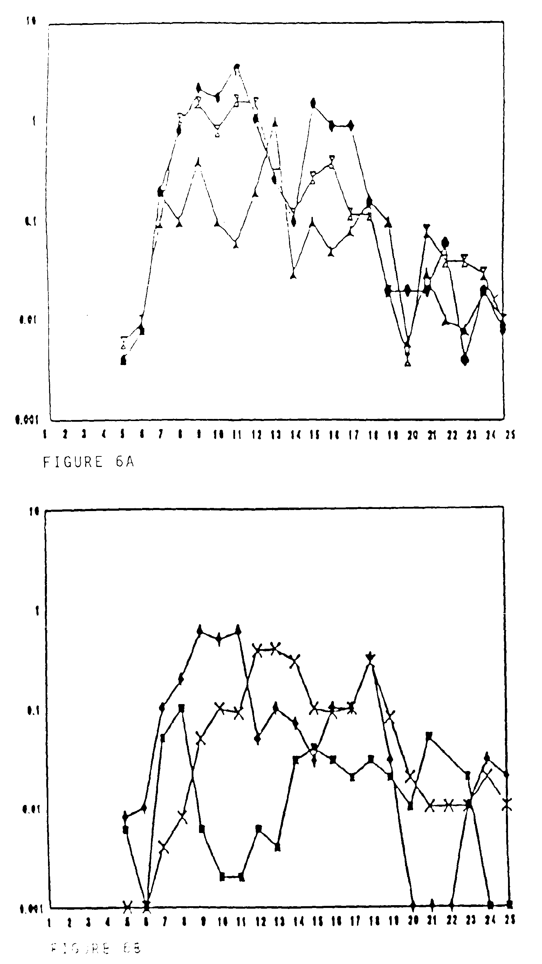

Les **Figures 6A-6G** sont illustratives des résultats obtenus. Dans chacune d'elles apparaissent les parasitémies (exprimées en pourcentages d'hématies parasitées sur l'axe des ordonnées à l'échelle logarithmique) observées chez les animaux d'épreuve en fonction des temps après l'infection (en jours sur les axes des abscisses).

Les résultats concernent :
- dans la **figure 6A** ; des animaux contrôles non vaccinés ;
- la **figure 6B** concerne des animaux qui avaient reçu une solution saline contenant en outre l'adjuvant de Freund ;

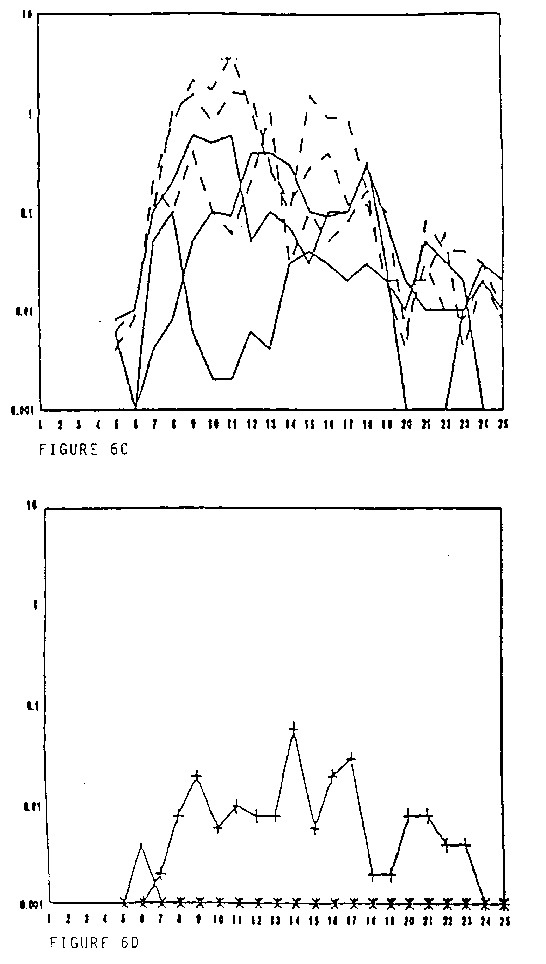

- la **figure 6C** est une superposition des figures 6A et 6B, dans le but de faire apparaître les résultats relatifs résultant de l'administration de l'adjuvant de Freund aux animaux (les variations ne sont évidemment pas significatives) ;
- la **figure 6D** fournit les résultats obtenus à l'issue d'une vaccination avec la p42 ;

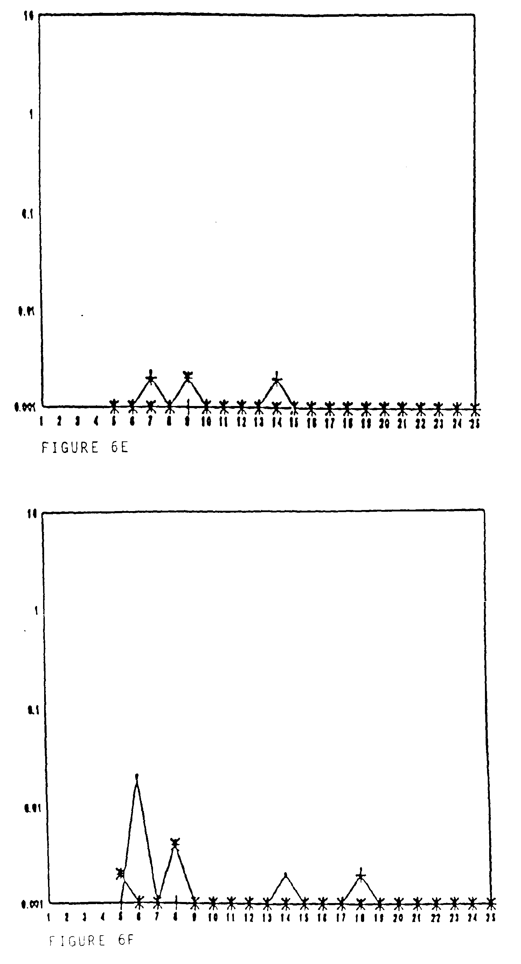

- la **figure 6E** concerne des animaux vaccinés avec la seule p19 ;
- enfin, la figure 6F concerne les animaux vaccinés avec un mélange de p19 et p42.

La p42 induit certes un certain niveau de protection. Mais comme en témoignent les figures 6E et 6F, la protection conférée par la p19 recombinante selon l'invention est considérablement améliorée.

On peut formuler l'hypothèse que l'amélioration de la protection résulte d'un clivage secondaire de la p42 qui s'accompagne du dévoilement de cystéine libre qui forme, par la suite, des ponts disulfure intermoléculaires donnant lieu à des multimères du p19 très caractéristiques de cette forme dans les protéines recombinantes des trois espèces testées.

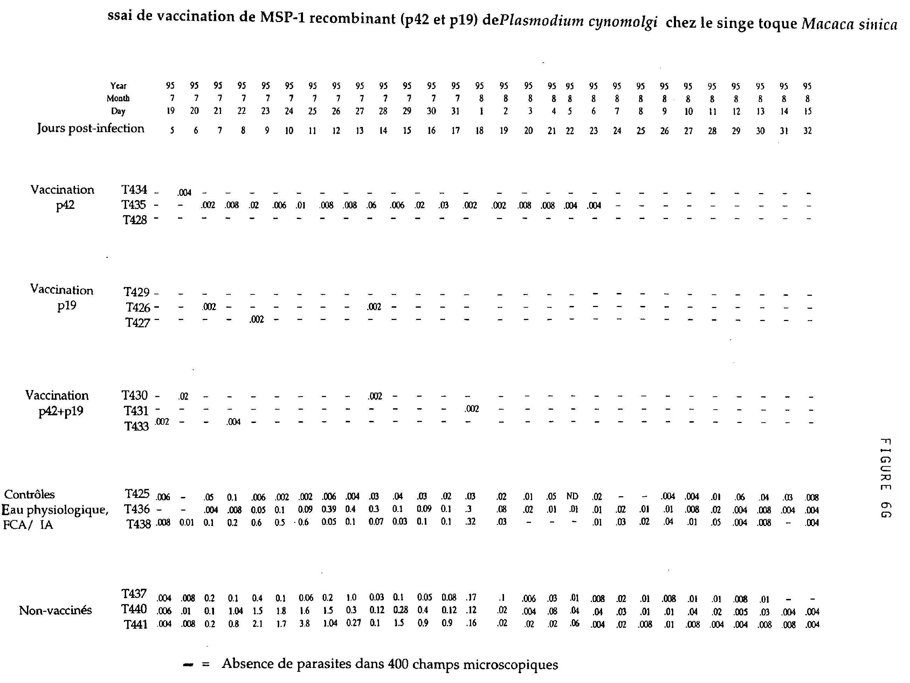

Les chiffres utilisés pour élaborer les graphiques (6A-6F) sont précisés dans la Figure 6G.

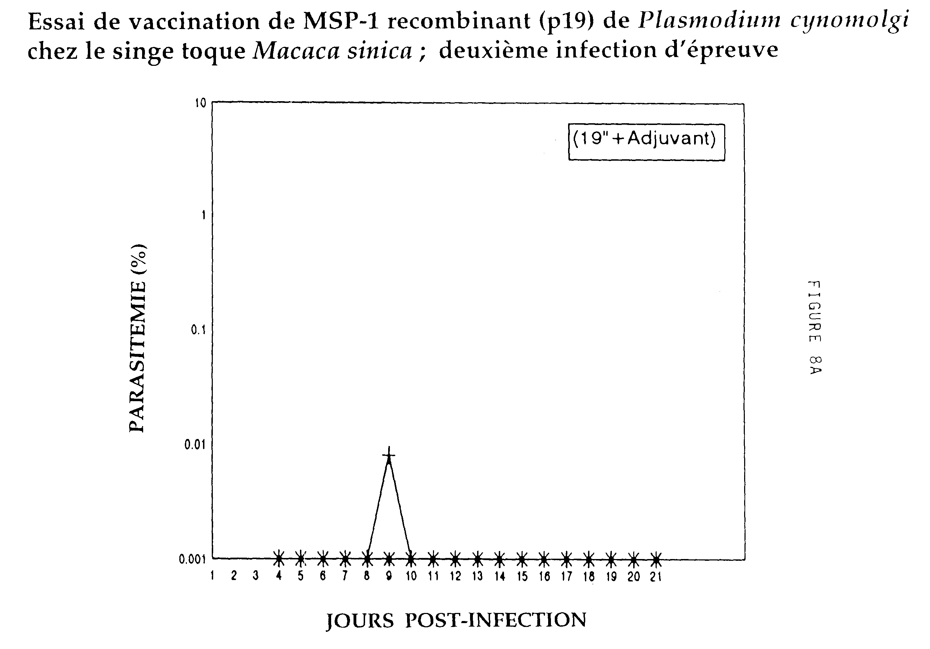

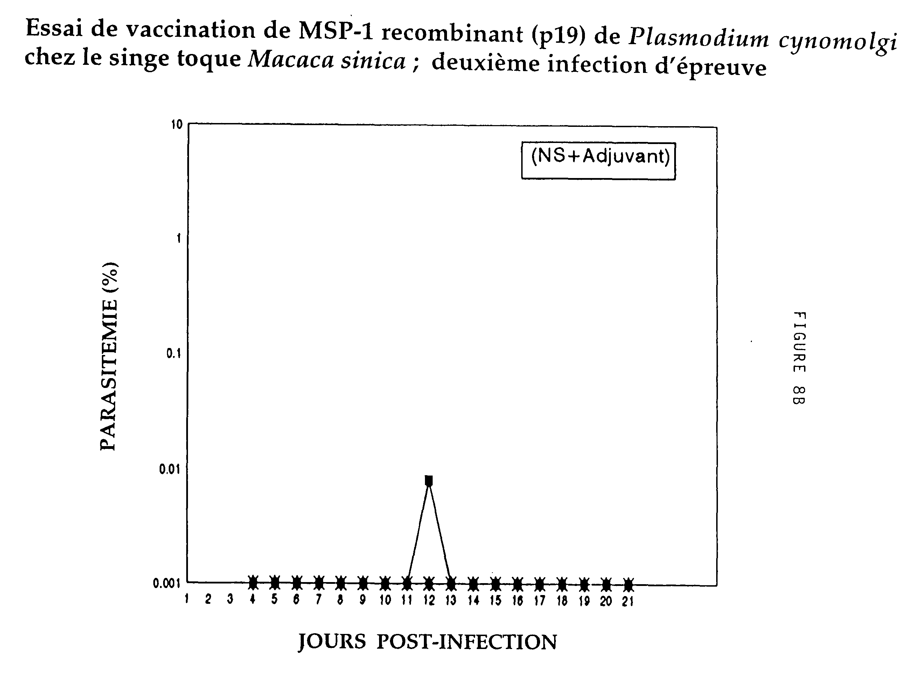

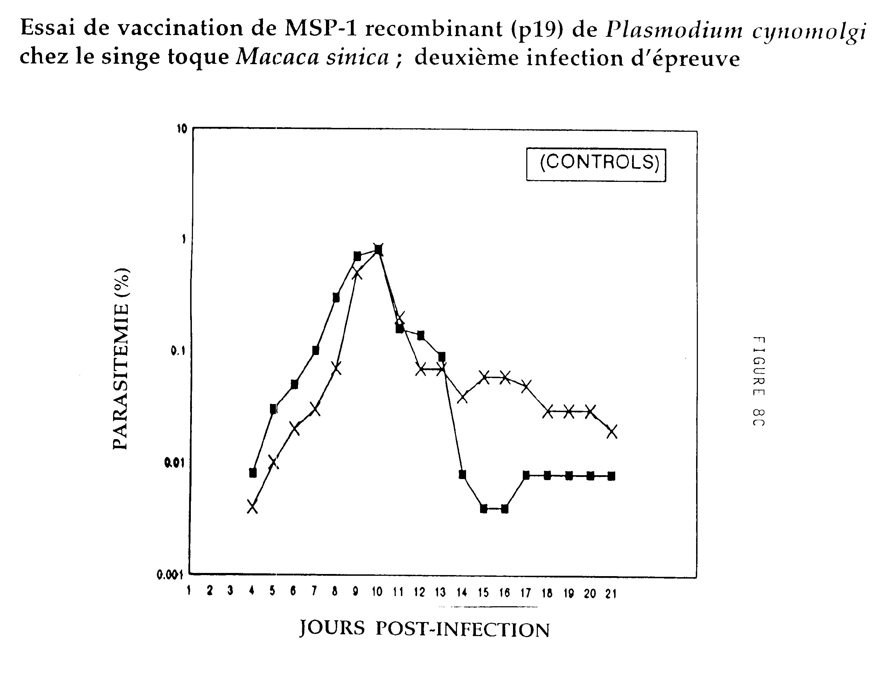

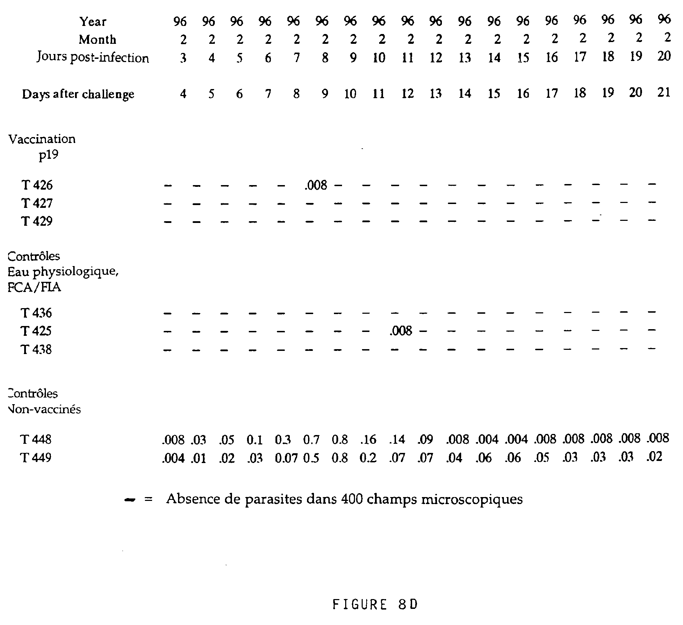

### Essai de vaccination P.cynomolgi/singe toque ; deuxième infection d'épreuve sur singes vaccinés avec p19 seule et contrôles (Figures 8)

Six mois plus tard, sans autre immunisation, les 3 singes ayant reçu le MSP-1 p19 seule avec FCA/FIA (Figure 6E) et les 3 singes ayant reçu une solution saline contenant l'adjuvant de Freund (Figure 6B) ainsi que 2 nouveaux singes naïfs non vaccinés ont subi une nouvelle infection d'épreuve par injection de 1.10⁶ hématies infectées avec *Plasmodium cynomolgi*. La protection a été évaluée en déterminant les parasitémies journalières chez tous les animaux en examinant les frottis avec du giemsa. Les parasitémies ont été classées comme négatives uniquement après comptage de 400 champs de frottis. Les parasitémies sont exprimées en pourcentage d'hématies parasitées (les chiffres utilisés pour élaborer les graphiques 8A-C sont précisés dans la **Figure 8D**). Les six animaux immunisés qui avaient subi une infection d'épreuve six mois auparavant n'avaient pas de parasitémie détectable sauf pour 1 animal dans chaque groupe qui a présenté une parasitémie à 0.008% pendant 1 jour (**Figures 8A** **et** **8B**). Les deux contrôles naïfs montrent une parasitémie classique avec un maximum de 0.8% et pendant 21 jours (**Figure 8C**) Donc, les 3 animaux vaccinés avec le MSP-1 p19 étaient aussi protégés six mois plus tard que les 3 contrôles qui présentaient une infection complète classique après le première infection d'épreuve, malgré une absence ou un très faible parasitémie après la première infection d'épreuve Ces résultats suggèrent que la durée de protection du p19 est au moins de six mois.

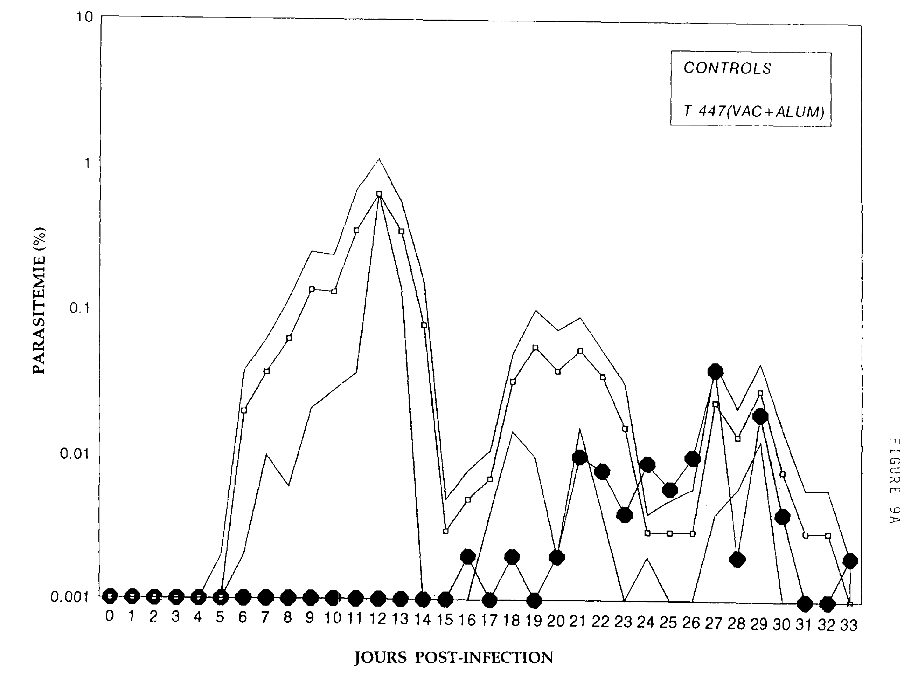

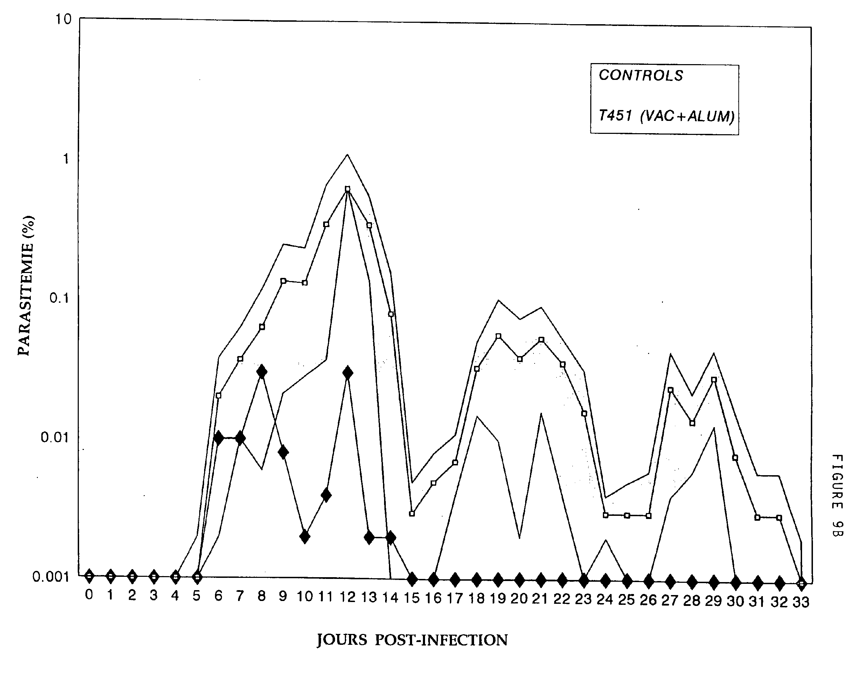

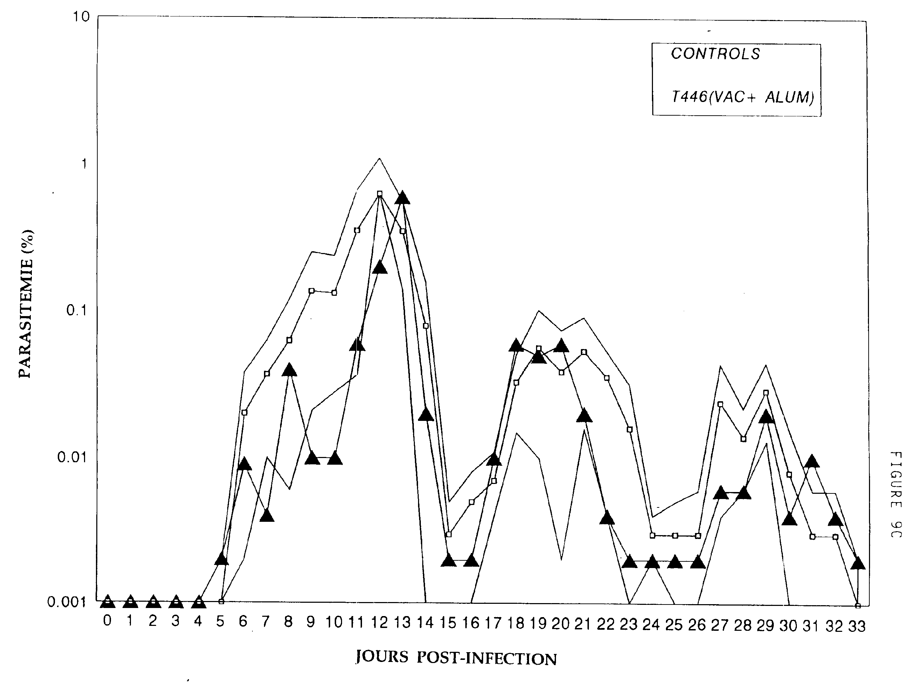

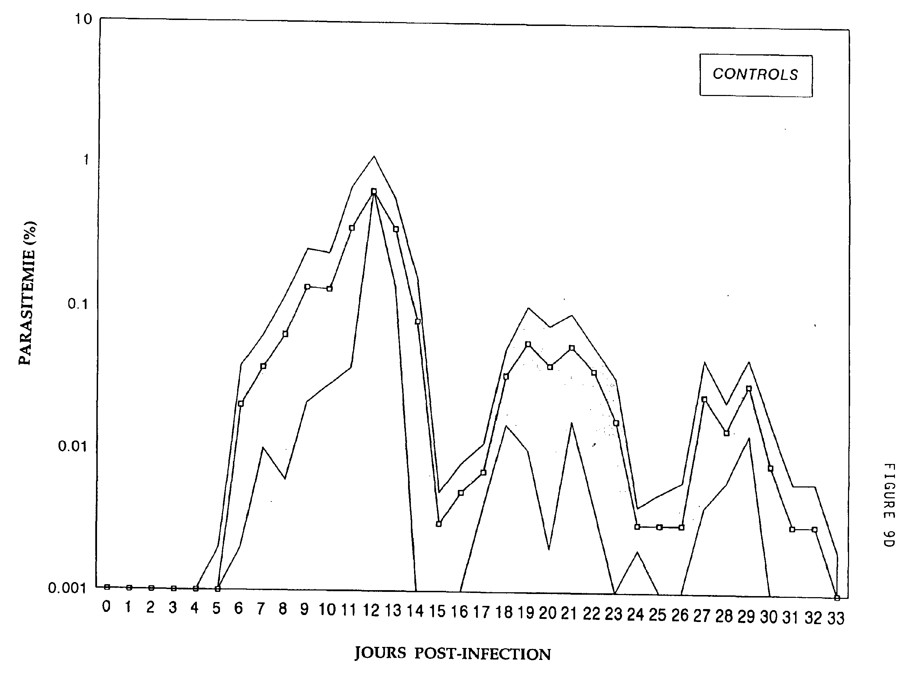

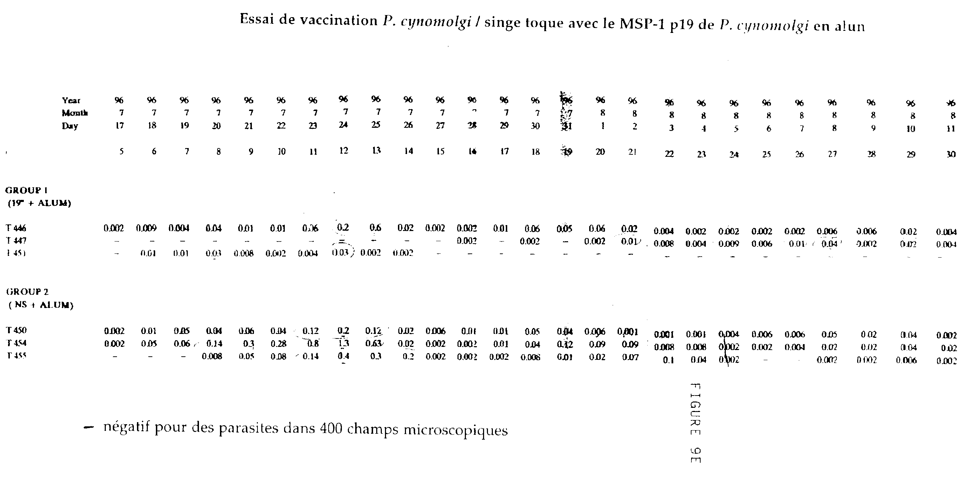

### Essai de vaccination avec p19 en association avec alun dans le système P.cynomolgi/singe toque (Figures 9)

Les résultats positifs de protection précédents ont été obtenus en utilisant l'adjuvant complet (FCA) ou incomplet (FIA) de Freund. Cependant, le seul adjuvant admis actuellement chez l'homme est l'alun. Pour cette raison, nous avons fait un essai de vaccination avec le MSP-1 p19 de P. *cynomolgi* chez le singe toque en présence d'alun comme adjuvant. Six singes capturés ont été utilisés comme suit: (1) 3 animaux injectés avec 4 doses de 50 mg MSP-1 p19 recombinant de *P. cynomolgi* avec 20 mg d'alun (2) 3 animaux injectés 4 fois avec de l'eau physiologique et 10 mg de alun. Les injections ont été faites par voie intramusculaire à 4 semaines d'intervalle. L'infection d'épreuve était faite en injectant 2.10⁵ hématies infectées avec P. *cynomolgi* 4 semaines après la dernière injection. La protection a été évaluée en déterminant les parasitémies journalières chez tous les animaux en examinant les frottis avec le giemsa. Les parasitémies ont été classées comme négatives uniquement après comptage de 400 champs de frottis. Les parasitémies sont exprimées en pourcentage d'hématies parasitées. Les résultats de cette expérience sont les suivants: 2 des 3 singes immunisés avec le p19 recombinant en alun avaient environ 30 fois moins de parasitémie totale pendant la durée de l'infection (**Figures 9A** **et** **9B**) que les 3 singes contrôles immunisés avec de l'eau physiologique et de l'alun (**Figure 9D**) après l'infection d'épreuve. Le troisième singe immunisé avec p19 (**Figure 9C**) n'était pas très différent des contrôles. Pour l'essai de vaccination de *Plasmodium cynomolgi* p19 chez le singe toque, *Macaca sinica*, décrit dans la Figure 9, les chiffres utilisés pour élaborer les graphiques (9A-9D) sont précisés (**Figure 9E**). Bien que ces résultats soient un peu moins spectaculaires que les précédents (Figures 6, 8) ; c'est la première fois qu'une protection significative est observée pour MSP-1 recombinante en alun.

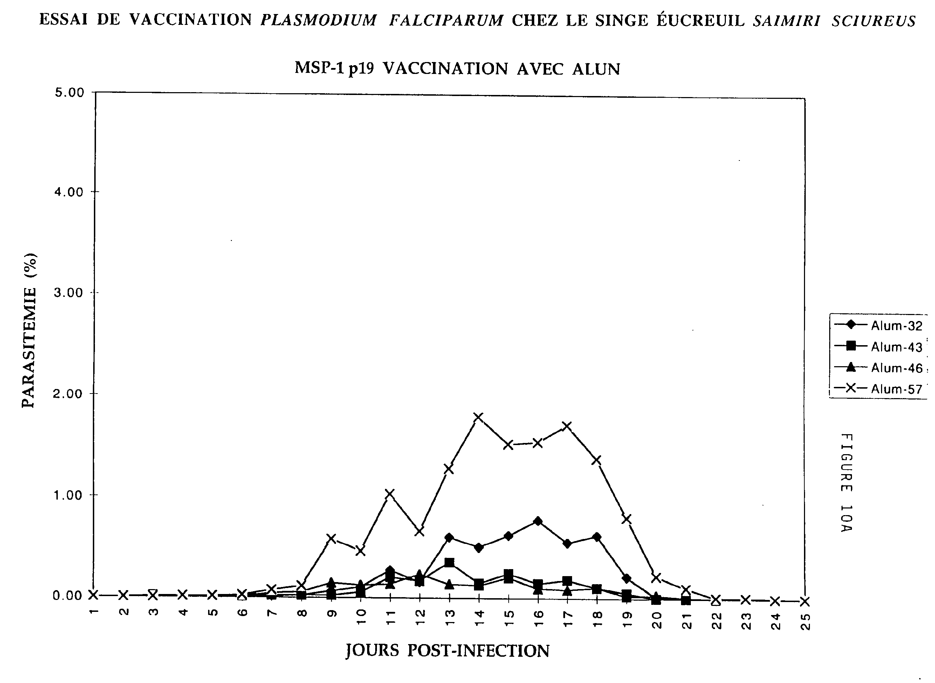

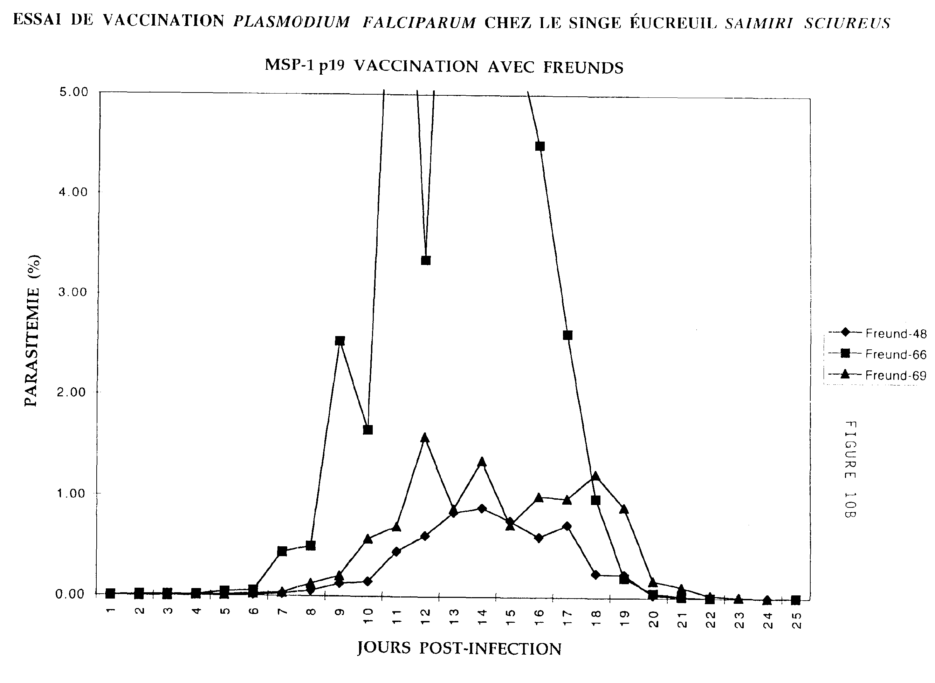

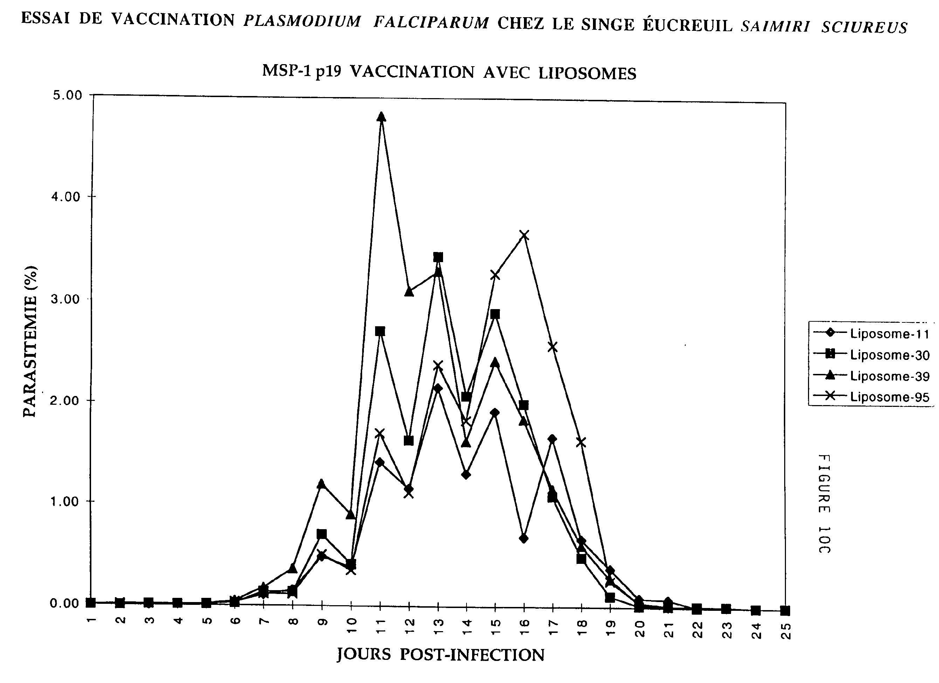

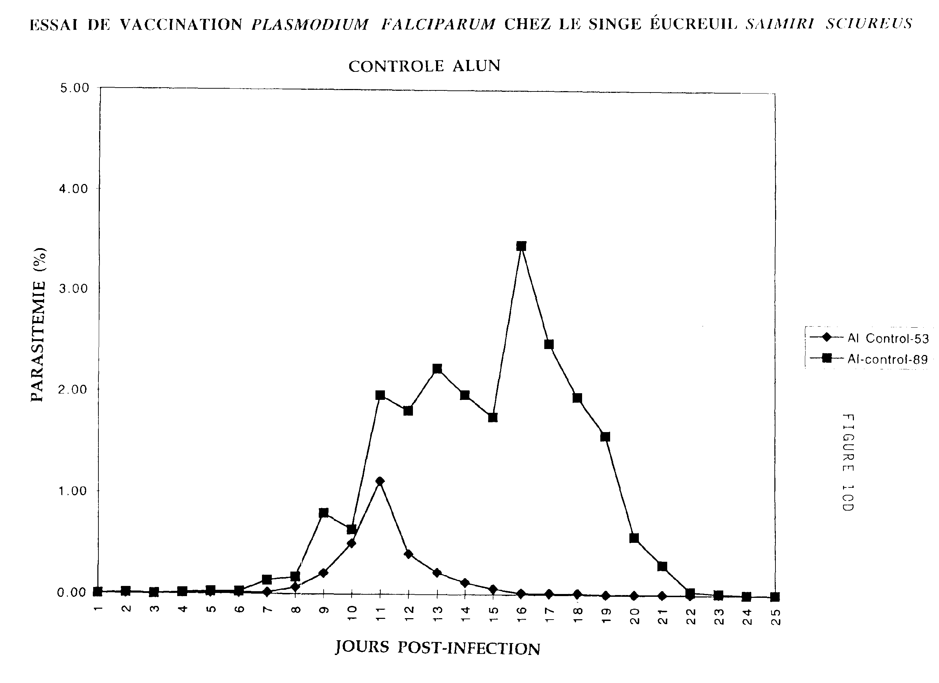

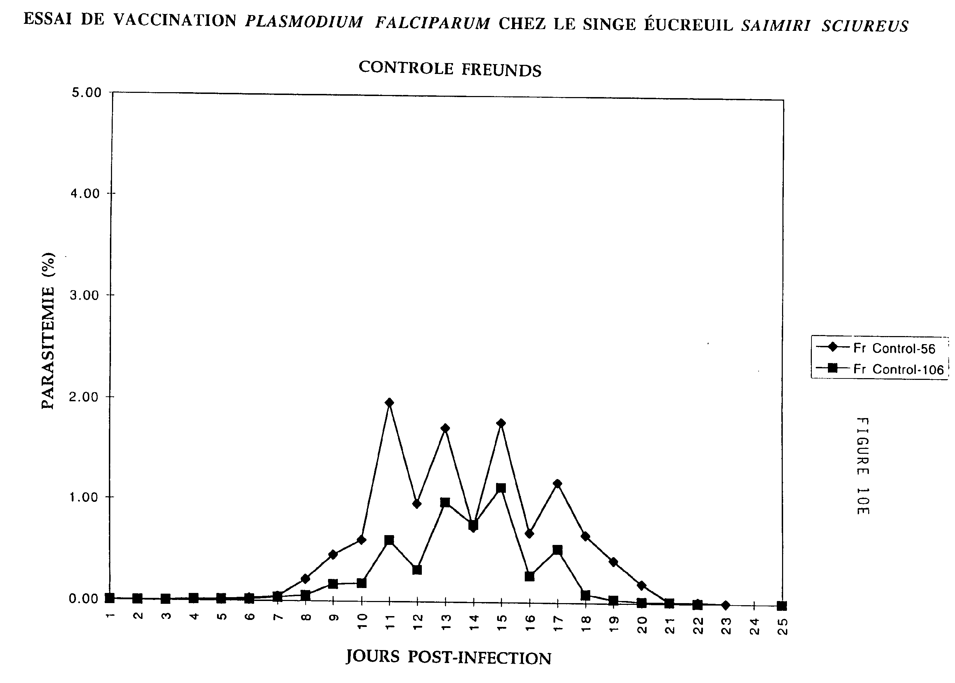

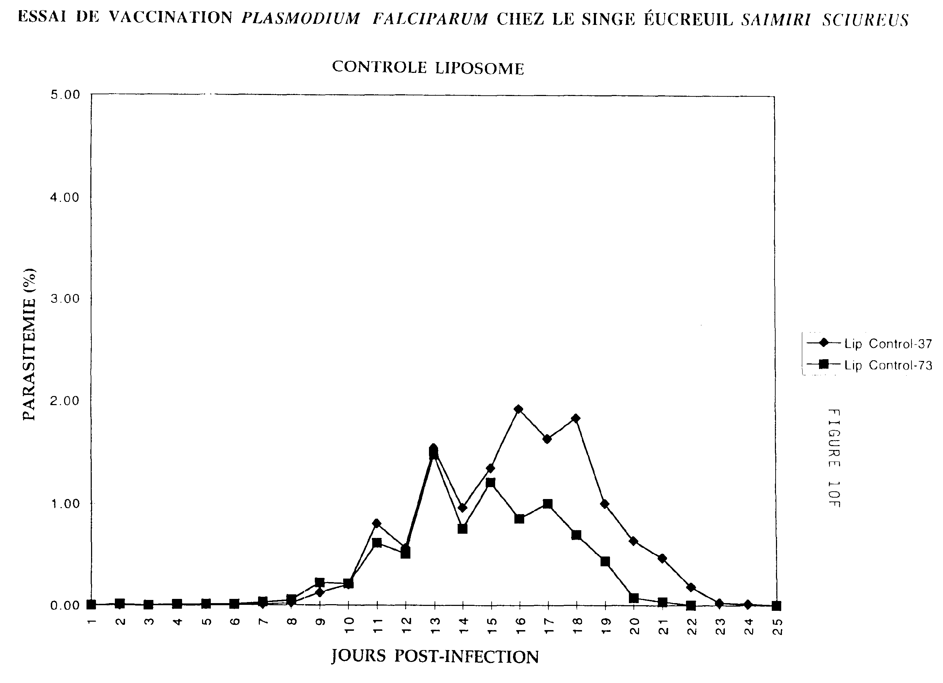

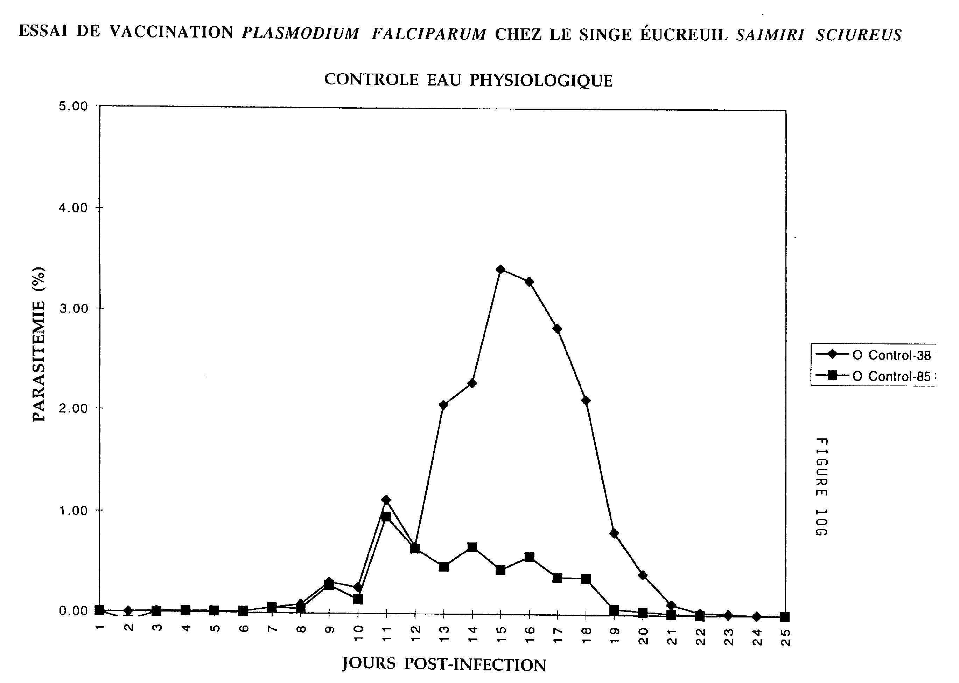

### Figure 10 : Essai de vaccination avec une p19 recombinante de Plasmodium falciparum chez le singe écureuil

Vingt singes *Saïmiri sciureus guyanensis* (singe écureuil) d'environ 3 ans élevés en captivité ont été utilisés comme suit: (1) 4 animaux injectés avec 50 mg de Pf MSP-1 p19 soluble en présence d'adjuvant de Freund comme suit: 1^{ère} injection: 1:1 FCA/FIA; 2^{ème} injection: 1:4 FCA/FIA; 3^{ème} injection: FIA Ces compositions d'adjuvant ont ensuite été mélangées avec l'antigène en PBS 1:1. (2) 2 animaux contrôles recevaient l'adjuvant de Freund comme décrit pour (1) avec uniquement PBS; (3) 4 animaux injectés avec 50 mg de Pf MSP-1 p19 soluble en présence de 10 mg d'alun (Alu-Gel-S, Serva); (4) 2 animaux contrôles recevaient 10 mg d'alun avec uniquement PBS; (5) 4 animaux injectés avec environ 50-100 mg Pf MSP-1 p19 ancré GPI reconstitués en liposomes comme suit: 300 mmoles de cholestérol et 300 mmoles de phosphatidyl choline étaient séchés sous vide et resuspendus en 330 mM N-octylglucoside en PBS avec 1.4 mg de Pf MSP-1 p19,GPI. Cette solution avait été dialysée contre PBS avec des Bio-Beads SM-2 adsorbant (Bio-Rad) et les liposomes ainsi formés étaient concentrés par centrifugation et resuspendus en PBS La 1^{ère} injection était faite avec des liposomes frais maintenus à 4°C et les 2^{éme} et 3^{éme} injections étaient faites avec des liposomes ayant été congelés pour conservation; (6) 2 animaux injectés avec des liposomes contrôles faits de la même façon en absence de l'antigène p19, GPI, comme décrit pour (5); (7) 2 animaux injectés avec de l'eau physiologique Trois injections ont été faites par voie intramusculaire à 4 semaines d'intervalle L'infection d'épreuve était faite en injectant 1.10⁶ hématies infectées avec *Plasmodium falciparum.* La protection a été évaluée en déterminant les parasitémies journalières chez tous les animaux en examinant les frottis avec giemsa. Les parasitémies sont exprimées en pourcentage d'hématies parasitées Les résultats de cet essai de vaccination sont exposés dans les **Figures 10****, A-G.**

Les groupes immunisés avec le p19 en adjuvant de Freund ou en liposome ont démontré des parasitémies semblables aux groupes contrôles après une infection d'épreuve (un animal (numéro 29) vacciné avec p19 en adjuvant de Freund est mort quelques jours après l'infection d'épreuve pour des raisons indépendantes de la vaccination (crise cardiaque)). Des irrégularités dans l'administration de l'antigène dans ces 2 groupes (mauvaise émulsion de Freund, liposomes congelés) ne permettent pas d'évaluer de façon complète la signification de ces résultats. Dans le groupe alun 2 animaux ont montré des parasitémies totales pendant la durée de l'infection environ 4 fois moins importantes que les contrôles, I animal environ 3 fois moins importante et 1 animal était semblable aux contrôles. Cette expérience est un peu difficile à interpréter à cause de la variabilité dans les contrôles, probablement due à la souche de parasite utilisée pour l'infection d'épreuve qui n'aurait pas été assez adaptée au modèle *Saimiri* non spénectomisé mis au point que récemment à Cayenne. Néanmoins, l'effet réel, quoiqu'imparfait, avec l'alun est encourageant dans la mesure où nos antigènes semblent être les seules versions recombinantes MSP-1 de *P*. *falciparum* qui, pour l'instant, ont démontré une certaine efficacité en association avec l'alun.

### Essai de vaccination avec une p19 recombinante de Plasmodium falciparum chez le singe écureuil (même essai que Figures 10)

Des singes élevés en captivité ont été injectés avec 1 ml d'inoculum par voie intramusculaire 2 fois à 4 semaines d'intervalle comme suit : **(1)** 4 animaux injectés avec 50 µg de PfMSP1p19 soluble en présence d'adjuvant de Freund comme suit: I^{ère} injection 1:1 FCA/FIA; 2^{ème} injection 1:4 FCA/FIA; et mélangés par la suite 1:1 avec l'antigène en PBS; **(2)** 4 animaux injectés avec 50 µg de PfMSP1p19 soluble en présence de 10 mg d'alun; **(3)** 4 animaux injectés avec environ 50 µg de PfMSP1p19 ancré GPI reconstitués en liposomes composés 1 1 en molarité de cholestérol et phosphatidyl choline. Les animaux ont été saignés 17 jours après la deuxième injection

Les globules rouges provenants d'un singe écureuil avec 30% de parasitémie due à *P. falciparum* (avec des formes mûres en majorité) ont été lavés en PBS et le culot était dilué 8 fois en présence de 2% SDS et 2% dithiothreitol et chauffé à 95° avant d'être chargé sur un gel de polyacrylamide de 7.5% (gel de séparation) et 4% (gel de stacking) (haut du gel) Après transfère en nitrocellulose l'analyse par immunoblot (immunoempreinte) a été fait avec des antisera comme suit: **(1)** pool d'antisera des 4 singes vaccinés avec PfMSP1p19 soluble en adjuvant de Freund dilué au vingtième ; **(2)** pool d'antisera des 4 singes vaccinés avec PfMSP1p19 soluble en adjuvant alun dilué au vingtième ; **(3)** pool d'antisera des 4 singes vaccinés avec PfMSP1p19 ancré en liposomes dilué au vingtième ; **(4)** l'anticorps monoclonal, qui réagit avec un épitope linéaire de PfMSP1p19, à 50 µg/ml ; **(5)** pool d'antisera SHI90 provenant d'un vingtaine de singes infectés à répétition par *P. falciparum* et devenus réfractaires à toute infection ultérieure de *P. falciparum,* dilué au cinq centième , **(6)** pool d'antisera des singes naïfs (n'ayant jamais été exposés *à P. falciparum*) dilué au vingtième.

Les résultats montrent que les 3 pools d'antisera des singes vaccinés avec le PfMSP1p19 réagissent de façon importante et spécifique avec des complexes de très haut poids moléculaire (se trouvant de façon diffuse dans le gel de stacking) et présents dans des extraits de parasite contenant davantage de formes mûres Ces résultats confortent l'hypothèse de la présence d'une agrégation spécifique du MSP1p19 *in vivo* comportant des épitopes qui sont reproduits dans les molécules recombinantes PfMSP1p19 synthétisées dans le système baculovirus, en particulier celles en forme d'oligomère.

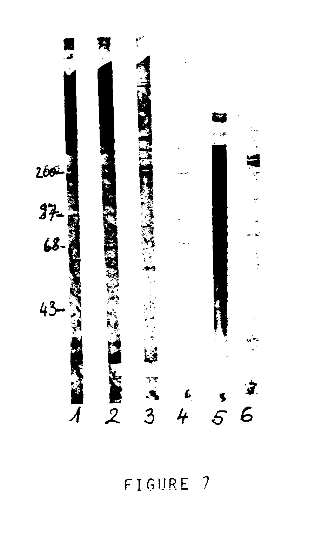

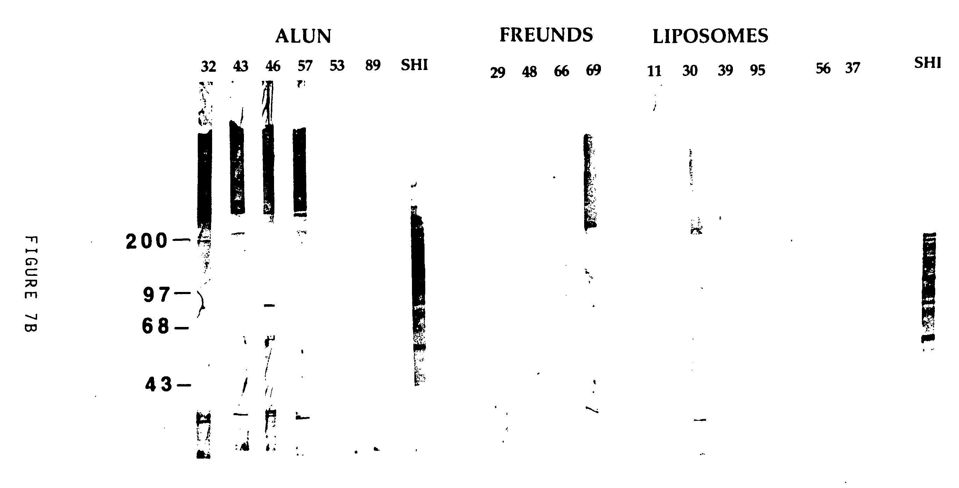

La **figure 7** illustre également ces résultats. Elle se rapporte aux immunoempreintes produites sur gel. Les trois premières colonnes du gel illustrent la réponse in vivo de singes à des injections de p19 [(1) avec l'adjuvant de Freund, (2) avec de l'alun, (3) sous forme de liposome] et notamment l'existence de complexes de haut poids moléculaire confortant l'hypothèse de l'agrégation in vivo de p19 sous forme d'oligomère, spécifique du stade de maturation (quand p42 est coupé en p19 et p33).

Cet essai de vaccination comprend également une troisième injection identique aux précédentes. L'injection avec l'adjuvant de Freund comprend uniquement du FIA.

Il y a deux animaux contrôles pour chaque groupe, à savoir : 2 animaux contrôles injectés avec PBS et l'adjuvant de Freund ; 2 animaux contrôles injectés avec PBS et alun ; 2 animaux contrôles injectés avec liposomes sans protéine ; et deux animaux injectés avec PBS sans adjuvant. La protection est évaluée comme décrit ci-dessus.

**Figure 7B****:** Les données pour cette figure sont dérivées de l'essai de vaccination de *P. falciparum* / singe écureuil (Figure 10 ci-après). Les chiffres correspondent aux singes individuels notés dans la Figure 10. Les techniques et méthodes pour cette figure sont les mêmes que pour la Figure 7 sauf que l'antisérum individuel de chaque singe noté a été testé après trois injections le jour de l'infection d'épreuve et l'antisérum SHI a été dilué 1:250. Les résultats montrent que l'antisérum des 4 singes vaccinés avec le p19 et alun réagissent de façon importante et spécifique avec des complexes de très haut poids moléculaire tandis que les singes des autres groupes vaccinés avec le p19 et l'adjuvant de Freund ou des liposomes ne montrent qu'une faible réactivité avec ces complexes. Puisque les singes vaccinés avec p19 et alun ont aussi été le mieux protégés, cette réactivité avec les complexes de haut poids moléculaire paraît indiquer un effet protecteur, et cela malgré qu'un singe dans le groupe alun n'était pas protégé par rapport aux contrôles et qu'un autre ne l'était que partiellement.

L'invention concerne naturellement d'autres applications, par exemple celles exposées ci-après en rapport avec certains des exemples, lesquels ne présentent aucun caractère limitatif.

### Thérapeutique

La molécule recombinante PfMSP1p19 peut être utilisée pour produire des anticorps spécifiques éventuellement utilisables par transfert passif dans un but de thérapeutique adaptée au paludisme sévère dû à P. *falciparum* avec risque de mortalité.

### Diagnostic

Les molécules recombinantes PvMSP1 p42 et PvMSP1p19 et PfMSP1p19 dérivées de baculovirus peuvent et ont été utilisées pour produire des anticorps monoclonaux spécifiques murins Ces anticorps, en combinaison avec des antisera polyclonaux anti MSP1p19 provenant d'une autre espèce telle que le lapin ou la chèvre, peuvent être à la base d'un test de diagnostic semi-quantitatif pour le paludisme et capable de distinguer entre un paludisme dû à P falciparum, qui peut être mortel, et un paludisme dû à P. vivax, qui n'est généralement pas mortel. Le principe de ce test serait de piéger et quantifier toute molécule de MSP1 contenant la partie p19 dans le sang.

Dans ce cadre, les avantages de la molécule MSP1p19 sont les suivants:
(i) elle est à la fois extrêmement bien conservée au sein d'une même espèce et suffisamment divergente entre des espèces différentes pour permettre de produire facilement des réactifs spécifiques d'espèce Aucune réaction croisée n'a été observée entre les anticorps dérivés de PfMSP1 p19 et PvMSP1p19 ;
(ii) la fonction du MSP1p19, bien que non connue avec précision, semble être suffisamment importante pour que cette molécule ne varie pas de façon significative ou soit délétée sans effet létal pour le parasite ;
(iii) c'est un antigène majeur se trouvant sur tous les mérozoïtes et donc, il doit être, en principe, détectable même à basse parasitémie et proportionnellement à la parasitémie ;
(iv) puisque les molécules recombinantes de MSP1p19 dérivées de baculovirus semblent reproduire davantage la structure native de MSP1p19, les anticorps produits contre ces protéines seraient bien adaptés à un usage diagnostique.

Les microorganismes identifiés ci-dessous ont été déposés suivant la règle 6.1. du Traité de Budapest à la date du 01 février 1996, sous les numéros suivants :

| Références d'identification | Numéros d'enregistrement |
|---|---|
| PvMSP1p19A | I - 1659 |
| PvMSP1p19S | I - 1660 |
| PfMSP1p19A | I - 1661 |
| PfMSP1p19S | I - 1662 |
| PcMSP1p19S | I - 1663 |

L'invention concerne également l'utilisation de ces anticorps, alors de préférence préalablement fixés sur un support solide (par exemple pour chromatographie d'affinité), pour la purification de peptides du type p19 initialement contenus dans un mélange.

La purification fait alors intervenir une mise en contact de ce mélange avec l'anticorps, la dissociation du complexe antigène-anticorps et la récupération du peptide de type p19 purifié.

L'invention concerne également des compositions de vaccin, comprenant également des mélanges de protéines ou de fragments, notamment des mélanges du type :

p19 de *P.falciparum* et p19 de *P. vivax*.
- p19 de *P.falciparum* et p42 de *P.falciparum,* celle-ci étant le cas échéant dépourvue de ses régions les plus hypervariables,
- p19 de *P.vivax* et p42 de *P.vivax,* celle-ci étant le cas échéant dépourvue de ses régions les plus hypervariables,
- p19 de *P.falciparum* et p42 de *P.falciparum,* celle-ci étant le cas échéant dépourvue de ses régions les plus hypervariables, et p19 de *P.vivax* et p42 de *P. vivax*, celle-ci étant le cas échéant dépourvue de ses régions les plus hypervariables.

On entend dans le cas présent définir les régions les plus hypervariables comme la région II ou la région II et la région III en partie ou dans sa totalité, la partie de la région III préférablement délétée étant celle qui est juxtaposée à la région II (la partie conservée se situant du côté C-terminal de la p33, proche de la p19). Les régions II et III sont illustrées en figure 4.

L'invention n'est pas limitée à la production de vaccin humain. Elle s'applique tout autant à la production de compositions de vaccin vétérinaire mettant en oeuvre les protéines ou antigènes correspondants dérivés de parasites infectieux pour des mammifères et produits dans les mêmes conditions. Il est en effet connu que des infections du même type, la babésiose, apparaissent aussi chez les bovins, les canins et les équins, un des antigènes des espèces Babésia présente une forte homologie conformationnelle (notamment les deux domaines « EFG-like » et richesse en cystéine) et fonctionnelle avec une partie protéique de MSP-1 [(36), (37) et (38)].

Des exemples de vaccins vétérinaires utilisant un antigène soluble contre de tels parasites on été décrits (39).

Il va sans dire que les p19 mises en oeuvre dans ces mélanges peuvent également donner lieu à toutes les modifications dont il a été question dans ce qui précède, lorsqu'elle était prise en considération de façon isolée.

### RÉFÉRENCES

**(1)** Holder, J A et al. (1982) « Biosynthesis and processing of a Plasmodium falciparum schizont antigen recognized by immune serum and a monoclonal antibody ». J. Exp. Med. 156 :1528-1538.
**(2)** Howard, R. et al. (1984) « Localization of the major Plasmodium falciparum glycoprotein on the surface of mature intracellular trophozoïtes and schizonts ». Mol. Biochem. Parasitol. 11 : 349-362.
**(3)** Pirson, P et al. (1985) « Characterization with monoclonal antibodies of a surface antigen of Plasmodium falciparum merozoïtes ». J. Immunol. 134 1946-1951
**(4)** Aley, S B et al. (1987) « Plasmodium vivax: Exoerythrocytic schizonts recognized by monoclonal antibodies against blood-stage schizonts ». Exp. Parasitol. 64 : 188-194.
**(5)** Holder, A A. (1988) « The precursor to major merozoïte surface antigen: structure and role in immunity ». Prog. Allergy 41 : 72-97.
**(6)** Cooper, J.A (1993) « Merozoïte surface antigen-1 of Plasmodium » Parasitol. Today 9 : 50-54.
**(7)** Holder, A.A., et al. (1987) « Processing of the precursor to the major merozoïte antigens of Plasmodium falciparum » Parasitology 94 : 199-208.
**(8)** Lyon, J.A. et al. (1986) « Epitope map and processing scheme for the 195 000-dalton surface glycoprotein of Plasmodium falciparum merozoïtes deduced from cloned overlapping segments of the gene ». Proc. Natl. Acad. Sci. USA 83 : 2989-2993.
**(9)** Blackman, M.J. et al (1992) « Secondary processing of the Plasmodium falciparum merozoïte surface protein-1 (MSP1) by calcium-dependent membrane-bound serine protease: shedding of MSP133 as a noncovalently associated complex with other fragments of the MSOP1 ». Mol Biochem. Parasitol. 50 : 307-316.
**(10)** Haldar, K , et al. (1985) « Acylation of a Plasmodium falciparum merozoïte surface antigen via sn-1,2-diacyl glycerol. ». J Biol. Chem, 260 : 4969-4974
**(11)** Braun Breton, C et al. (1990) « Glycolipid anchorage of Plasmodium falciparum surface antigens ». Res. Immunol 141 : 743-755.
**(12)** Kumar, S. et al. (1995) « Immunogenicity and in vivo Efficacy of Recombinant Plasmodium falciparum Merozoïte surface protein-1 in Aotus Monkeys ». Molecular Medicine, Vol. 1, 3 : 325-332.
**(14)** Longacre, S. et al. (1994) « Plasmodium vivax merozoïte surface protein 1 C-terminal recombinant proteins in baculovirus ». Mol. Biochem. Parasitol. 64 : 191-205.
**(15)** McBride, J.S. et al. (1987) « Fragments of the polymorphic Mr 185 000 glycoprotein from the surface of isolated Plasmodium falciparum merozoïtes form an antigenic complex ». Mol. Biochem. Parasitol. 23 : 71-84.
**(16)** Blackman, M.J. et al. (1990) « A single fragment of a malaria merozoïte surface protein remains on the parasite during red cell invasion and is the target of invasion-inhibiting antibodies ». J. Exp. Med. 172 : 379-382.
**(17)** Kaslow, D.C. et al. (1994) « Expression and antigenicity of Plasmodium falciparum major merozoïte surface protein (MSP119) variants secreted from Saccharomyces cerevisiae ». Mol. biochem. Parasitol. 63 ; 283-289.
**(18)** Chang, S.P., et al. (1992) « A carboxyl-terminal fragment of Plasmodium falciparum gp195 expressed by a recombinant baculovirus induces antibodies that completely inhibit parasite growth ». J. Immunol. 149 : 548-555.
**(19)** Longacre, S. (1995) « The Plasmodium cynomolgi merozoïte surface protein 1 C-terminal sequence and its homologies with other Plasmodium species ». Mol. Biochem. Parasitol. 74 :105-111.
**(20)** Del Portillo, H.A., et al. (1990) « Primary structure of the merozoïte surface antigen 1 of Plasmodium vivax reveals sequences conserved between différent Plasmodium species ». Proc. Natl. Acad. Sci. USA 88 : 4030-4034.
**(21)** Gibson, H.L., et al. (1992) « Structure and expression of the gene for Pv200, a major blood-stage surface antigen of Plasmodium vivax ». Mol. biochem. Parasitol. 50 : 325-334.
**(22)** Dissanaike, A.S., et al. (1965) « Two new malaria parasites, Plasmodium cynomolgi ceylonensis sub sp. nov. and Plasmodium fragile sp. nov. from monkeys in Ceylon ». Ceylon Journal of Medical Science 14 : 1-9.
**(23)** Cochrane, A.H., et al (1986) « Further studies on the antigenic diversity of the circumsporozoïte proteins of the Plasmodium cynomolgi complex ». Am J. Trop. Med. Hyg. 35 : 479-487.
**(24)** Naotunne, T de S , et al. (1990) « Plasmodium cynomolgi: serum-mediated blocking and enhancement of infectivity to mosquitos during infections in the natural host, Macaca sinica » Exp. Parasitol. 71, 305-313.
**(25)** Ihalamulla, R.L. et al. (1987) « Plasmodium vivax: isolation of mature asexual stages and gametocytes from infected human blood by colloidal silica (Percoll) gradient centrifugation » Trans R. Soc. Trop. Med. Hyg. 81 : 25-28
**(26)** Kimura, E., et al. (1990) « Genetic diversity in the major merozoite surface antigen of Plasmodium falciparum high prevalence of a third polymorphic form detected in strains derived in strains derived from malaria patients ». Gene 91 : 57-62.
**(27)** Heidrich, H.-G., et al. (1989) « The N-terminal amino acid sequences of the Plasmodium falciparum (FCBI) merozoïte surface antigen of 42 and 36 kilodalton, both derived from the 185-195 kilodalton precursor ». Mol. Biochem. Parasitol. 34 : 147-154.
**(28)** Blackman M.J., et al (1991) « Proteolytic processing of the Plasmodium falciparum merozoïte surface protein-1 produces a membrane-bound fragment containing two epidermal growth factor-like domains ». Mol. Biochem. Parasitol. 49 : 29-34.
**(29)** Adams. J. M. et al (1992) « A family of erythrocyte binding proteins of malaria parasites ». Proc. Natl. Acad Sci, 89:7085-7089
**(30)** Sim B.K.L (1995) « EBA-175 An erythrocyte-binding ligand of Plasmodium falciparum » Parasitology Today, vol.II, n°6:213-217.
**(31)** Sim B K L (1994) « Receptor and ligand domains for invasion of erythrocytes by Plasmodium falciparum ». Science, 264:1941-1944.
**(32)** Davies, A et al (1993), Biochem J. 295 (Pt3) : 889-896 « Expression of the glycosylphosphatidylinositol-linked complement-inhibiting protein CD59 antigen in insect cells using a baculovirus vector ».
**(33)** Haziot A. et al. (1994) J. Immunol. 152 : 5868. « Recombinant soluble CD14 Inhibits LPS-Induced Tumor Necrosis Factor & Production by Cells in Whole Blood ».
**(34)** Chang, S.P. et al., (1988) « Plasmodium falciparum : gene structure and hydropathy profile of the major merozoite surface antigen (gp195) of the Uganda-Palo Alto isolate. Exp. Parasitol. 67 : 1-11.
**(35)** Holder, A.S. et al. (1985) « Primary structure of the precursor to the three major surface antigens of Plasmodium falciparum merozoites, Nature 317 : 270-273.
**(36)** G. Bourdoiseau et al. (mai 1995) « Les babésioses bovines », Le point vétérinaire, vol.27, n°168.
**(37)** P. Bourdeau et al. (mai 1995) « La babésiose canine à Babesia canis », Le point vétérinaire, vol.27, n°168.
**(38)** C. Soulé (mai 1995) « Les babésioses équines », Le point vétérinaire, vol.27, n°168.
**(39)** T.P.M. Schetters et al. (1995) « Vaccines against Babesiosis using Soluble Parasite Antigens », Parasitology Today, vol.11, n°12.
**(40)** P.A. Burghaus et al. (1996) « Immunization of Aotus nancymai with Recombinant C Terminus of Plasmodium falciparum Merozoite Surface Protein 1 in Liposomes and Alun Adjuvant Does Not Induce Protection against a Challenge Infection », Infection and Immunity, 64:3614-3619.
**(41)** S.P. Chang, et al. (1996) « A Recombinant Baculovirus 42-Kilodalton C-Terminal Fragment of Plasmodium falciparum Merozoite Surface Protein 1 Protects Aotus Monkeys against Malaria », Infection and Immunity, 64: 253-261.
**(42)** L.H. Miller et al. (1997) « The Need for Assays Predictive of Protection in Development of Malaria Bloodstage Vaccines », Parasitology Today, vol. 13, n°2:46-47.

L'invention concerne également les hybridomes sécréteurs d'anticorps spécifiques reconnaissant sélectivement la p19 d'une protéine MSP-1 de la forme mérozoïte d'un parasite du type *Plasmodium* infectieux pour l'homme autre que *Plasmodium vivax* et ne reconnaissant pas *Plasmodium vivax.*

En particulier, ces hybridomes sécrètent des anticorps monoclonaux qui reconnaissent pas la p19 de *Plasmodium vivax et* qui reconnaissent spécifiquement la p19 de *Plasmodium falciparum.*

L'invention concerne également un hybridome caractérisé en ce qu'il produit un anticorps spécifique qui reconnaît spécifiquement la p19 de *P vivax* et la p19 de *P cynomolgi*. Un hybridome F10-3 a été construit à partir du myélome X63 Ag8 653 produisant des IgG 2b/k reconnaissant la glycoprotéine p42 de *Plasmodium vivax*.

LISTE DE SEQUENCES
(1) INFORMATIONS GENERALES:
(i) DEPOSANT:
(A) NOM: INSTITUT PASTEUR
(B) RUE: 25-28, rue du Docteur Roux
(C) VILLE: PARIS
(E) PAYS: FRANCE
(F) CODE POSTAL: 75724 CEDEX 15
(G) TELEPHONE: 0145688093
(H) TELECOPIE: 0140613017
(A) NOM: NEW YORK UNIVERSITY
(B) RUE: MEDICAL CENTER - 550 First Avenue
(C) VILLE: NEW YORK
(D) ETAT OU PROVINCE: NEW YORK
(E) PAYS: ETATS-UNIS D'AMERIQUE
(F) CODE POSTAL: 10016
(ii) TITRE DE L' INVENTION: PROTEINE RECOMBINANTE CONTENANT UN FRAGMENT
C-TERMINAL DE LA PROTEINE MSP-1 D'UN PLASMODIUM INFECTIEUX
POUR DES MAMMIFERES, NOTAMMENT L'HOMME POUR LA PRODUCTION
DE VACCINS ANTI-PALUDIQUES
(iii) NOMBRE DE SEQUENCES: 14
(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)
(v) DONNEES DE LA DEMANDE ACTUELLE:
NUMERO DE LA DEMANDE: PCT/FR 97/00290
(vi) DONNEES DE LA DEMANDE ANTERIEURE:
(A) NUMERO DE LA DEMANDE: FR 9601822
(B) DATE DE DEPOT: 14-FEB-1996
(2) INFORMATIONS POUR LA SEQ ID NO: 1:
(i) CARACTERISTIQUES DE LA SEQUENCE:
(A) LONGUEUR: 291 paires de bases
(B) TYPE: nuclEotide
(C) NOMBRE DE BRINS: simple
(D) CONFIGURATION: linEaire
(ii) TYPE DE MOLECULE: Autre acide nuclEique
(A) DESCRIPTION: /desc = "FIGURE 1A"
(ix) CARACTERISTIQUE:
(A) NOM/CLE: CDS
(B) EMPLACEMENT:1__291
(D) AUTRES INFORMATIONS:/label= Bac19
(xi) DESCRIPTION DE LA SEQUENCE: SEQ ID NO: 1:
GAA TTC AAC ATC TCG CAG CAC CAA TGC GTG AAA AAA CAA TGT CCC GAG 48
Glu Phe Asn Ile Ser Gln His Gln Cys Val Lys Lys Gln Cys Pro Glu
1               5                   10                  15
AAC TCT GGC TGT TTC AGA CAC TTG GAC GAG AGA GAG GAG TGT AAA TGT 96
Asn Ser Gly Cys Phe Arg His Leu Asp Glu Arg Glu Glu Cys Lys Cys
            20                  25                  30
CTG CTG AAC TAC AAA CAG GAG GGC GAC AAG TGC GTG GAG AAC CCC AAC 144
Leu Leu Asn Tyr Lys Gln Glu Gly Asp Lys Cys Val Glu Asn Pro Asn
        35                  40                  45
CCG ACC TGT AAC GAG AAC AAC GGC GGC TGT GAC GCA GAC GCC AAA TGC 192
Pro Thr Cys Asn Glu Asn Asn Gly Gly Cys Asp Ala Asp Ala Lys Cys
    50                  55                  60
ACC GAG GAG GAC TCG GGC AGC AAC GGC AAG AAA ATC ACG TGT GAG TGT 240
Thr Glu Glu Asp Ser Gly Ser Asn Gly Lys Lys Ile Thr Cys Glu Cys
65                  70                  75                  80
ACC AAA CCC GAC TCG TAC CCG CTG TTC GAC GGC ATC TTC TGC AGC TAA 288
Thr Lys Pro Asp Ser Tyr Pro Leu Phe Asp Gly Ile Phe Cys Ser *
                85                  90                  95
TAA 291
*
(2) INFORMATIONS POUR LA SEQ ID NO: 2:
(i) CARACTERISTIQUES DE LA SEQUENCE:
(A) LONGUEUR: 95 acides aminEs
(B) TYPE: acide aminE
(D) CONFIGURATION: linEaire

(ii) TYPE DE MOLECULE: protEine
(xi) DESCRIPTION DE LA SEQUENCE: SEQ ID NO: 2:
Glu Phe Asn Ile Ser Gln His Gln Cys Val Lys Lys Gln Cys Pro Glu
1               5                   10                  15
Asn Ser Gly Cys Phe Arg His Leu Asp Glu Arg Glu Glu Cys Lys Cys
            20                  25                  30
Leu Leu Asn Tyr Lys Gln Glu Gly Asp Lys Cys Val Glu Asn Pro Asn
        35                  40                  45
Pro Thr Cys Asn Glu Asn Asn Gly Gly Cys Asp Ala Asp Ala Lys Cys
    50                  55                  60
Thr Glu Glu Asp Ser Gly Ser Asn Gly Lys Lys Ile Thr Cys Glu Cys
65                  70                  75                  80
Thr Lys Pro Asp Ser Tyr Pro Leu Phe Asp Gly Ile Phe Cys Ser
                85                  90                  95
(2) INFORMATIONS POUR LA SEQ ID NO: 3:
(i) CARACTERISTIQUES DE LA SEQUENCE:
(A) LONGUEUR: 279 paires de bases
(B) TYPE: nuclEotide
(C) NOMBRE DE BRINS: simple
(D) CONFIGURATION: linEaire
(ii) TYPE DE MOLECULE: Autre acide nuclEique
(A) DESCRIPTION: /desc = "FIGURE 1A"
(ix) CARACTERISTIQUE:
(A) NOM/CLE: misc_feature
(B) EMPLACEMENT:1__279
(D) AUTRES INFORMATIONS:/label= PF19
(xi) DESCRIPTION DE LA SEQUENCE: SEQ ID NO: 3:
AACATTTCAC AACACCAATG CGTAAAAAAA CAATGTCCAG AAAATTCTGG ATGTTTCAGA 60
CATTTAGATG AAAGAGAAGA ATGTAAATGT TTATTAAATT ACAAACAAGA AGGTGATAAA 120
TGTGTTGAAA ATCCAAATCC TACTTGTAAC GAAAATAATG GTGGATGTGA TGCAGATGCC 180
AAATGTACCG AAGAAGATTC AGGTAGCAAC GGAAAGAAAA TCACATGTGA ATGTACTAAA 240
CCTGATTCTT ATCCACTTTT CGATGGTATT TTCTGCAGT 279
(2) INFORMATIONS POUR LA SEQ ID NO: 4:
(i) CARACTERISTIQUES DE LA SEQUENCE:
(A) LONGUEUR: 354 paires de bases
(B) TYPE: nuclEotide
(C) NOMBRE DE BRINS: simple
(D) CONFIGURATION: linEaire
(ii) TYPE DE MOLECULE: Autre acide nuclEique
(A) DESCRIPTION: /desc = "FIGURE 1B"
(ix) CARACTERISTIQUE:
(A) NOM/CLE: CDS
(B) EMPLACEMENT:1__354
(D) AUTRES INFORMATIONS:/label= Bac19
(xi) DESCRIPTION DE LA SEQUENCE: SEQ ID NO: 4:
GAA TTC AAC ATC TCG CAG CAC CAA TGC GTG AAA AAA CAA TGT CCC GAG 48
Glu Phe Asn Ile Ser Gln His Gln Cys Val Lys Lys Gln Cys Pro Glu
100 105 110
AAC TCT GGC TGT TTC AGA CAC TTG GAC GAG AGA GAG GAG TGT AAA TGT 96
Asn Ser Gly Cys Phe Arg His Leu Asp Glu Arg Glu Glu Cys Lys Cys
115 120 125
CTG CTG AAC TAC AAA CAG GAG GGC GAC AAG TGC GTG GAG AAC CCC AAC 144
Leu Leu Asn Tyr Lys Gln Glu Gly Asp Lys Cys Val Glu Asn Pro Asn
130 135 140 145
CCG ACC TGT AAC GAG AAC AAC GGC GGC TGT GAC GCA GAC GCC AAA TGC 192
Pro Thr Cys Asn Glu Asn Asn Gly Gly Cys Asp Ala Asp Ala Lys Cys
150 155 160
ACC GAG GAG GAC TCG GGC AGC AAC GGC AAG AAA ATC ACG TGT GAG TGT 240
Thr Glu Glu Asp Ser Gly Ser Asn Gly Lys Lys Ile Thr Cys Glu Cys
165 170 175
ACC AAA CCC GAC TCG TAC CCG CTG TTC GAC GGC ATC TTC TGC AGC TCC 288
Thr Lys Pro Asp Ser Tyr Pro Leu Phe Asp Gly Ile Phe Cys Ser Ser
180 185 190
TCT AAC TTC TTG GGC ATC TCG TTC TTG TTG ATC CTC ATG TTG ATC TTG 336
Ser Asn Phe Leu Gly Ile Ser Phe Leu Leu Ile Leu Met Leu Ile Leu
195 200 205
TAC AGC TTC ATT TAA TAA 354
Tyr Ser Phe Ile * *
210 215

( 2) I NFORMATI ONS POUR LA SEQ I D NO: 5:
( i ) CARACTERI STI QUES DE LA SEQUENCE:
( A) LONGUEUR: 116 aci des ami nEs
( B) TYPE: aci de ami nE
( D) CONFI GURATI ON: l i nEai r e
( i i ) TYPE DE MOLECULE: pr ot Ei ne
( xi ) DESCRI PTI ON DE LA SEQUENCE: SEQ I D NO: 5:
Gl u Phe Asn I l e Ser Gl n Hi s Gl n Cys Val Lys Lys Gl n Cys Pr o Gl u
1 5 10 15
Asn Ser Gl y Cys Phe Ar g Hi s Leu Asp Gl u Ar g Gl u Gl u Cys Lys Cys
20 25 30
Leu Leu Asn Tyr Lys Gl n Gl u Gl y Asp Lys Cys Val Gl u Asn Pr o Asn
35 40 45
Pr o Thr Cys Asn Gl u Asn Asn Gl y Gl y Cys Asp Al a Asp Al a Lys Cys
50 55 60
Thr Gl u Gl u Asp Ser Gl y Ser Asn Gl y Lys Lys I l e Thr Cys Gl u Cys
65 70 75 80
Thr Lys Pr o Asp Ser Tyr Pr o Leu Phe Asp Gl y I l e Phe Cys Ser Ser
85 90 95
Ser Asn Phe Leu Gl y I l e Ser Phe Leu Leu I l e Leu Met Leu I l e Leu
100 105 110
Tyr Ser Phe I l e
115
( 2) I NFORMATI ONS POUR LA SEQ I D NO: 6:
( i ) CARACTERI STI QUES DE LA SEQUENCE:
( A) LONGUEUR: 342 pai r es de bases
( B) TYPE: nucl Eot i de
( C) NOMBRE DE BRI NS: si mpl e
( D) CONFI GURATI ON: l i nEai r e
( i i ) TYPE DE MOLECULE: Aut r e aci de nucl Ei que
( A) DESCRI PTI ON: / desc = " FI GURE 1B"
( i x) CARACTERI STI QUE:
( A) NOM/ CLE: mi sc_f eat ur e
( B) EMPLACEMENT: 1__342
( D) AUTRES I NFORMATI ONS: / l abel = PF19
( xi ) DESCRI PTI ON DE LA SEQUENCE: SEQ I D NO: 6:
AACATTTCAC AACACCAATG CGTAAAAAAA CAATGTCCAG AAAATTCTGG ATGTTTCAGA 60
CATTTAGATG AAAGAGAAGA ATGTAAATGT TTATTAAATT ACAAACAAGA AGGTGATAAA 120
TGTGTTGAAA ATCCAAATCC TACTTGTAAC GAAAATAATG GTGGATGTGA TGCAGATGCC 180
AAATGTACCG AAGAAGATTC AGGTAGCAAC GGAAAGAAAA TCACATGTGA ATGTACTAAA 240
CCTGATTCTT ATCCACTTTT CGATGGTATT TTCTGCAGTT CCTCTAACTT CTTAGGAATA 300
TCATTCTTAT TAATACTCAT GTTAATATTA TACAGTTTCA TT 342
( 2) I NFORMATI ONS POUR LA SEQ I D NO: 7:
( i ) CARACTERI STI QUES DE LA SEQUENCE:
( A) LONGUEUR: 387 pai r es de bases
( B) TYPE: nucl Eot i de
( C) NOMBRE DE BRI NS: si mpl e
( D) CONFI GURATI ON: l i nEai r e
( i i ) TYPE DE MOLECULE: Aut r e aci de nucl Ei que
( A) DESCRI PTI ON: / desc = " FI GURE 1C"
( i x) CARACTERI STI QUE:
( A) NOM/ CLE: CDS
( B) EMPLACEMENT: 1__387
( xi ) DESCRI PTI ON DE LA SEQUENCE: SEQ I D NO: 7:
ATG AAG GCG CTA CTC TTT TTG TTC TCT TTC ATT TTT TTC GTT ACC AAA 48
Met Lys Al a Leu Leu Phe Leu Phe Ser Phe I l e Phe Phe Val Thr Lys
120 125 130
TGT CAA TGT GAA ACA GAA AGT TAT AAG CAG CTT GTA GCC AAC GTG GAC 96
Cys Gl n Cys Gl u Thr Gl u Ser Tyr Lys Gl n Leu Val Al a Asn Val Asp
135 140 145 150
GAA TTC AAC ATC TCG CAG CAC CAA TGC GTG AAA AAA CAA TGT CCC GAG 144
Gl u Phe Asn I l e Ser Gl n Hi s Gl n Cys Val Lys Lys Gl n Cys Pr o Gl u
155 160 165
AAC TCT GGC TGT TTC AGA CAC TTG GAC GAG AGA GAG GAG TGT AAA TGT 192
Asn Ser Gl y Cys Phe Ar g Hi s Leu Asp Gl u Ar g Gl u Gl u Cys Lys Cys
170 175 180
CTG CTG AAC TAC AAA CAG GAG GGC GAC AAG TGC GTG GAG AAC CCC AAC 240
Leu Leu Asn Tyr Lys Gl n Gl u Gl y Asp Lys Cys Val Gl u Asn Pr o Asn
185 190 195

CCG ACC TGT AAC GAG AAC AAC GGC GGC TGT GAC GCA GAC GCC AAA TGC 288
Pro Thr Cys Asn Glu Asn Asn Gly Gly Cys Asp Ala Asp Ala Lys Cys
200 205 210
ACC GAG GAG GAC TCG GGC AGC AAC GGC AAG AAA ATC ACG TGT GAG TGT 336
Thr Glu Glu Asp Ser Gly Ser Asn Gly Lys Lys Ile Thr Cys Glu Cys
215 220 225 230
ACC AAA CCC GAC TCG TAC CCG CTG TTC GAC GGC ATC TTC TGC AGC TAA 384
Thr Lys Pro Asp Ser Tyr Pro Leu Phe Asp Gly Ile Phe Cys Ser *
235 240 245
TAA 387
*
(2) INFORMATIONS POUR LA SEQ ID NO: 8:
(i) CARACTERISTIQUES DE LA SEQUENCE:
(A) LONGUEUR: 127 acides aminEs
(B) TYPE: acide aminE
(D) CONFIGURATION: linEaire
(ii) TYPE DE MOLECULE: protEine
(xi) DESCRIPTION DE LA SEQUENCE: SEQ ID NO: 8:
Met Lys Ala Leu Leu Phe Leu Phe Ser Phe Ile Phe Phe Val Thr Lys
1               5                   10                  15
Cys Gln Cys Glu Thr Glu Ser Tyr Lys Gln Leu Val Ala Asn Val Asp
            20                  25                  30
Glu Phe Asn Ile Ser Gln His Gln Cys Val Lys Lys Gln Cys Pro Glu
        35                  40                  45
Asn Ser Gly Cys Phe Arg His Leu Asp Glu Arg Glu Glu Cys Lys Cys
    50                  55                  60
Leu Leu Asn Tyr Lys Gln Glu Gly Asp Lys Cys Val Glu Asn Pro Asn
65                  70                  75                  80
Pro Thr Cys Asn Glu Asn Asn Gly Gly Cys Asp Ala Asp Ala Lys Cys
                85                  90                  95
Thr Glu Glu Asp Ser Gly Ser Asn Gly Lys Lys Ile Thr Cys Glu Cys
            100                 105                 110
Thr Lys Pro Asp Ser Tyr Pro Leu Phe Asp Gly Ile Phe Cys Ser
        115                 120                 125
(2) INFORMATIONS POUR LA SEQ ID NO: 9:
(i) CARACTERISTIQUES DE LA SEQUENCE:
(A) LONGUEUR: 330 paires de bases
(B) TYPE: nuclEotide
(C) NOMBRE DE BRINS: simple
(D) CONFIGURATION: linEaire
(ii) TYPE DE MOLECULE: Autre acide nuclEique
(A) DESCRIPTION: /desc = "FIGURE 1D"
(ix) CARACTERISTIQUE:
(A) NOM/CLE: CDS
(B) EMPLACEMENT:1__330
(xi) DESCRIPTION DE LA SEQUENCE: SEQ ID NO: 9:
GAA ACA GAA AGT TAT AAG CAG CTT GTA GCC AAC GTG GAC GAA TTC AAC 48
Glu Thr Glu Ser Tyr Lys Gln Leu Val Ala Asn Val Asp Glu Phe Asn
130 135 140 145
ATC TCG CAG CAC CAA TGC GTG AAA AAA CAA TGT CCC GAG AAC TCT GGC 96
Ile Ser Gln His Gln Cys Val Lys Lys Gln Cys Pro Glu Asn Ser Gly
150 155 160
TGT TTC AGA CAC TTG GAC GAG AGA GAG GAG TGT AAA TGT CTG CTG AAC 144
Cys Phe Arg His Leu Asp Glu Arg Glu Glu Cys Lys Cys Leu Leu Asn
165 170 175
TAC AAA CAG GAG GGC GAC AAG TGC GTG GAG AAC CCC AAC CCG ACC TGT 192
Tyr Lys Gln Glu Gly Asp Lys Cys Val Glu Asn Pro Asn Pro Thr Cys
180 185 190
AAC GAG AAC AAC GGC GGC TGT GAC GCA GAC GCC AAA TGC ACC GAG GAG 240
Asn Glu Asn Asn Gly Gly Cys Asp Ala Asp Ala Lys Cys Thr Glu Glu
195 200 205
GAC TCG GGC AGC AAC GGC AAG AAA ATC ACG TGT GAG TGT ACC AAA CCC 288
Asp Ser Gly Ser Asn Gly Lys Lys Ile Thr Cys Glu Cys Thr Lys Pro
210 215 220 225
GAC TCG TAC CCG CTG TTC GAC GGC ATC TTC TGC AGC TAA TAA 330
Asp Ser Tyr Pro Leu Phe Asp Gly Ile Phe Cys Ser * *
230 235
(2) INFORMATIONS POUR LA SEQ ID NO: 10:
(i) CARACTERISTIQUES DE LA SEQUENCE:

(A) LONGUEUR: 108 acides aminEs
(B) TYPE: acide aminE
(D) CONFIGURATION: linEaire
(ii) TYPE DE MOLECULE: protEine
(xi) DESCRIPTION DE LA SEQUENCE: SEQ ID NO: 10:
Glu Thr Glu Ser Tyr Lys Gln Leu Val Ala Asn Val Asp Glu Phe Asn
1               5                   10                  15
Ile Ser Gln His Gln Cys Val Lys Lys Gln Cys Pro Glu Asn Ser Gly
            20                  25                  30
Cys Phe Arg His Leu Asp Glu Arg Glu Glu Cys Lys Cys Leu Leu Asn
        35                  40                  45
Tyr Lys Gln Glu Gly Asp Lys Cys Val Glu Asn Pro Asn Pro Thr Cys
    50                  55                  60
Asn Glu Asn Asn Gly Gly Cys Asp Ala Asp Ala Lys Cys Thr Glu Glu
65                  70                  75                  80
Asp Ser Gly Ser Asn Gly Lys Lys Ile Thr Cys Glu Cys Thr Lys Pro
                85                  90                  95
Asp Ser Tyr Pro Leu Phe Asp Gly Ile Phe Cys Ser
100 105 110
(2) INFORMATIONS POUR LA SEQ ID NO: 11:
(i) CARACTERISTIQUES DE LA SEQUENCE:
(A) LONGUEUR: 379 acides aminEs
(B) TYPE: acide aminE
(C) NOMBRE DE BRINS: simple
(D) CONFIGURATION: linEaire
(ii) TYPE DE MOLECULE: peptide
(ix) CARACTERISTIQUE:
(A) NOM/CLE: Protein
(B) EMPLACEMENT:1__379
(D) AUTRES INFORMATIONS:/label= cynomolgi
/note= "FIGURE 4"
(ix) CARACTERISTIQUE:
(A) NOM/CLE: Region
(B) EMPLACEMENT:1__139
(D) AUTRES INFORMATIONS:/note= "REGION I"
(ix) CARACTERISTIQUE:
(A) NOM/CLE: Region
(B) EMPLACEMENT:140__177
(D) AUTRES INFORMATIONS:/note= "REGION II"
(ix) CARACTERISTIQUE:
(A) NOM/CLE: Region
(B) EMPLACEMENT:178__282
(D) AUTRES INFORMATIONS:/note= "REGION III"
(ix) CARACTERISTIQUE:
(A) NOM/CLE: Region
(B) EMPLACEMENT:283__379
(D) AUTRES INFORMATIONS:/note= "REGION IV"
(xi) DESCRIPTION DE LA SEQUENCE: SEQ ID NO: 11:
Asp Gln Val Thr Thr Gly Glu Ala Glu Ser Glu Ala Pro Glu Ile Ile
1               5                   10                  15
Val Pro Gln Gly Ile Asn Glu Tyr Asp Val Val Tyr Ile Lys Pro Leu
            20                  25                  30
Ala Gly Met Tyr Lys Thr Ile Lys Lys Pro Leu Glu Asn His Val Asn
        35                  40                  45
Ala Leu Asn Thr Asn Ile Ile Asp Met Leu Asp Ser Arg Leu Lys Lys
    50                  55                  60
Arg Asn Tyr Phe Leu Asp Val Leu Asn Ser Asp Leu Asn Pro Tyr Ser
65                  70                  75                  80
Ile Pro His Ser Gly Glu Tyr Ile Ile Lys Asp Pro Tyr Lys Leu Leu
                85                  90                  95
Asp Leu Glu Lys Lys Lys Leu Leu Gly Ser Tyr Lys Tyr Ile Gly Ala
            100                 105                 110
Ser Val Asp Lys Asp Met Val Thr Ala Asn Asp Gly Leu Ala Tyr Tyr
        115                 120                 125
Gln Lys Met Gly Asp Leu Tyr Lys Lys His Leu Asp Glu Val Asn Ala
    130                 135                 140
Cys Ile Lys Glu Val Glu Ala Asn Ile Asn Lys His Asp Glu Glu Ile
145                 150                 155                 160
Lys Lys Ile Gly Ser Glu Ala Ser Lys Ala Asn Asp Lys Asn Gln Leu

                165                 170                 175
Asn Ala Lys Lys Glu Glu Leu Gln Lys Tyr Leu Pro Phe Leu Ser Ser
            180                 185                 190
Ile Gln Lys Glu Tyr Ser Thr Leu Val Asn Lys Val His Ser Tyr Thr
        195                 200                 205
Asp Thr Leu Lys Lys Ile Ile Asn Asn Cys Gln Ile Glu Lys Lys Glu
    210                 215                 220
Thr Glu Thr Ile Val Asn Lys Leu Glu Asp Tyr Ser Lys Met Asp Glu
225                 230                 235                 240
Glu Leu Asp Val Tyr Lys Gln Ser Lys Lys Glu Asp Asp Val Lys Ser
                245                 250                 255
Ser Gly Leu Leu Glu Lys Leu Met Asn Ser Lys Leu Ile Asn Gln Glu
            260                 265                 270
Glu Ser Lys Lys Ala Leu Ser Glu Leu Leu Asn Val Gln Thr Gln Met
        275                 280                 285
Leu Asn Met Ser Ser Glu His Arg Cys Ile Asp Thr Asn Val Pro Glu
    290                 295                 300
Asn Ala Ala Cys Tyr Arg Tyr Leu Asp Gly Thr Glu Glu Trp Arg Cys
305                 310                 315                 320
Leu Leu Tyr Phe Lys Glu Asp Ala Gly Lys Cys Val Pro Ala Pro Asn
                325                 330                 335
Met Thr Cys Lys Asp Lys Asn Gly Gly Cys Ala Pro Glu Ala Glu Cys
            340                 345                 350
Lys Met Asn Asp Lys Asn Glu Ile Val Cys Lys Cys Thr Lys Glu Gly
        355                 360                 365
Ser Glu Pro Leu Phe Glu Gly Val Phe Cys Ser
    370                 375
(2) INFORMATIONS POUR LA SEQ ID NO: 12:
(i) CARACTERISTIQUES DE LA SEQUENCE:
(A) LONGUEUR: 380 acides aminEs
(B) TYPE: acide aminE
(C) NOMBRE DE BRINS: simple
(D) CONFIGURATION: linEaire
(ii) TYPE DE MOLECULE: peptide
(ix) CARACTERISTIQUE:
(A) NOM/CLE: Protein
(B) EMPLACEMENT:1__380
(D) AUTRES INFORMATIONS:/label= vivax Belem
/note= "FIGURE 4"
(ix) CARACTERISTIQUE:
(A) NOM/CLE: Region
(B) EMPLACEMENT:1__140
(D) AUTRES INFORMATIONS:/note= "REGION I"
(ix) CARACTERISTIQUE:
(A) NOM/CLE: Region
(B) EMPLACEMENT:141__178
(D) AUTRES INFORMATIONS:/note= "REGION II"
(ix) CARACTERISTIQUE:
(A) NOM/CLE: Region
(B) EMPLACEMENT:179__283
(D) AUTRES INFORMATIONS:/note= "REGION III"
(ix) CARACTERISTIQUE:
(A) NOM/CLE: Region
(B) EMPLACEMENT:284__380
(D) AUTRES INFORMATIONS:/note= "REGION IV"
(xi) DESCRIPTION DE LA SEQUENCE: SEQ ID NO: 12:
Asp Gln Val Thr Thr Gly Glu Ala Glu Ser Glu Ala Pro Glu Ile Leu
1               5                   10                  15
Val Pro Ala Gly Ile Ser Asp Tyr Asp Val Val Tyr Leu Lys Pro Leu
            20                  25                  30
Ala Gly Met Tyr Lys Thr Ile Lys Lys Gln Leu Glu Asn His Val Asn
        35                  40                  45
Ala Phe Asn Thr Asn Ile Thr Asp Met Leu Asp Ser Arg Leu Lys Lys
    50                  55                  60
Arg Asn Tyr Phe Leu Glu Val Leu Asn Ser Asp Leu Asn Pro Phe Lys
65                  70                  75                  80
Tyr Ser Pro Ser Gly Glu Tyr Ile Ile Lys Asp Pro Tyr Lys Leu Leu
                85                  90                  95
Asp Leu Glu Lys Lys Lys Lys Leu Leu Gly Ser Tyr Lys Tyr Ile Gly

            100                 105                 110
Ala Ser Ile Asp Lys Asp Leu Ala Thr Ala Asn Asp Gly Val Thr Tyr
        115                 120                 125
Tyr Asn Lys Met Gly Glu Leu Tyr Lys Thr His Leu Thr Ala Val Asn
    130                 135                 140
Glu Glu Val Lys Lys Val Glu Ala Asp Ile Lys Ala Glu Asp Asp Lys
145                 150                 155                 160
Ile Lys Lys Ile Gly Ser Asp Ser Thr Lys Thr Thr Glu Lys Thr Gln
                165                 170                 175
Ser Met Ala Lys Lys Ala Glu Leu Glu Lys Tyr Leu Pro Phe Leu Asn
            180                 185                 190
Ser Leu Gln Lys Glu Tyr Glu Ser Leu Val Ser Lys Val Asn Thr Tyr
        195                 200                 205
Thr Asp Asn Leu Lys Lys Val Ile Asn Asn Cys Gln Leu Glu Lys Lys
    210                 215                 220
Glu Ala Glu Ile Thr Val Lys Lys Leu Gln Asp Tyr Asn Lys Met Asp
225                 230                 235                 240
Glu Lys Leu Glu Glu Tyr Lys Lys Ser Glu Lys Lys Asn Glu Val Lys
                245                 250                 255
Ser Ser Gly Leu Leu Glu Lys Leu Met Lys Ser Lys Leu Ile Lys Glu
            260                 265                 270
Asn Glu Ser Lys Glu Ile Leu Ser Gln Leu Leu Asn Val Gln Thr Gln
        275                 280                 285
Leu Leu Thr Met Ser Ser Glu His Thr Cys Ile Asp Thr Asn Val Pro
    290                 295                 300
Asp Asn Ala Ala Cys Tyr Arg Tyr Leu Asp Gly Thr Glu Glu Trp Arg
305                 310                 315                 320
Cys Leu Leu Thr Phe Lys Glu Glu Gly Gly Lys Cys Val Pro Ala Ser
                325                 330                 335
Asn Val Thr Cys Lys Asp Asn Asn Gly Gly Cys Ala Pro Glu Ala Glu
            340                 345                 350
Cys Lys Met Thr Asp Ser Asn Lys Ile Val Cys Lys Cys Thr Lys Glu
        355                 360                 365
Gly Ser Glu Pro Leu Phe Glu Gly Val Phe Cys Ser
    370                 375                 380
(2) INFORMATIONS POUR LA SEQ ID NO: 13:
(i) CARACTERISTIQUES DE LA SEQUENCE:
(A) LONGUEUR: 380 acides aminEs
(B) TYPE: acide aminE
(C) NOMBRE DE BRINS: simple
(D) CONFIGURATION: linEaire
(ii) TYPE DE MOLECULE: peptide
(ix) CARACTERISTIQUE:
(A) NOM/CLE: Protein
(B) EMPLACEMENT:1__380
(D) AUTRES INFORMATIONS:/label= vivax Sal 1
/note= "FIGURE 4"
(ix) CARACTERISTIQUE:
(A) NOM/CLE: Region
(B) EMPLACEMENT:1__140
(D) AUTRES INFORMATIONS:/note= "REGION I"
(ix) CARACTERISTIQUE:
(A) NOM/CLE: Region
(B) EMPLACEMENT:141__178
(D) AUTRES INFORMATIONS:/note= "REGION II"
(ix) CARACTERISTIQUE:
(A) NOM/CLE: Region
(B) EMPLACEMENT:179__283
(D) AUTRES INFORMATIONS:/note= "REGION III"
(ix) CARACTERISTIQUE:
(A) NOM/CLE: Region
(B) EMPLACEMENT:284__380
(D) AUTRES INFORMATIONS:/note= "REGION IV"
(xi) DESCRIPTION DE LA SEQUENCE: SEQ ID NO: 13:
Asp Gln Val Thr Thr Gly Glu Ala Glu Ser Glu Ala Pro Glu Ile Leu
1               5                   10                  15
Val Pro Ala Gly Ile Ser Asp Tyr Asp Val Val Tyr Leu Lys Pro Leu
            20                  25                  30
Ala Gly Met Tyr Lys Thr Ile Lys Lys Gln Leu Glu Asn His Val Asn

        35                  40                  45
Ala Phe Asn Thr Asn Ile Thr Asp Met Leu Asp Ser Arg Leu Lys Lys
    50                  55                  60
Arg Asn Tyr Phe Leu Glu Val Leu Asn Ser Asp Leu Asn Pro Phe Lys
65                  70                  75                  80
Tyr Ser Ser Ser Gly Glu Tyr Ile Ile Lys Asp Pro Tyr Lys Leu Leu
                85                  90                  95
Asp Leu Glu Lys Lys Lys Lys Leu Ile Gly Ser Tyr Lys Tyr Ile Gly
            100                 105                 110
Ala Ser Ile Asp Met Asp Leu Ala Thr Ala Asn Asp Gly Val Thr Tyr
        115                 120                 125
Tyr Asn Lys Met Gly Glu Leu Tyr Lys Thr His Leu Asp Gly Val Lys
    130                 135                 140
Thr Glu Ile Lys Lys Val Glu Asp Asp Ile Lys Lys Gln Asp Glu Glu
145                 150                 155                 160
Leu Lys Lys Leu Gly Asn Val Asn Ser Gln Asp Ser Lys Lys Asn Glu
                165                 170                 175
Phe Ile Ala Lys Lys Ala Glu Leu Glu Lys Tyr Leu Pro Phe Leu Asn
            180                 185                 190
Ser Leu Gln Lys Glu Tyr Glu Ser Leu Val Ser Lys Val Asn Thr Tyr
        195                 200                 205
Thr Asp Asn Leu Lys Lys Val Ile Asn Asn Cys Gln Leu Glu Lys Lys
    210                 215                 220
Glu Ala Glu Ile Thr Val Lys Lys Leu Gln Asp Tyr Asn Lys Met Asp
225                 230                 235                 240
Glu Lys Leu Glu Glu Tyr Lys Lys Ser Glu Lys Lys Asn Glu Val Lys
                245                 250                 255
Ser Ser Gly Leu Leu Glu Lys Leu Met Lys Ser Lys Leu Ile Lys Glu
            260                 265                 270
Asn Glu Ser Lys Glu Ile Leu Ser Gln Leu Leu Asn Val Gln Thr Gln
        275                 280                 285
Leu Leu Thr Met Ser Ser Glu His Thr Cys Ile Asp Thr Asn Val Pro
    290                 295                 300
Asp Asn Ala Ala Cys Tyr Arg Tyr Leu Asp Gly Thr Glu Glu Trp Arg
305                 310                 315                 320
Cys Leu Leu Thr Phe Lys Glu Glu Gly Gly Lys Cys Val Pro Ala Ser
                325                 330                 335
Asn Val Thr Cys Lys Asp Asn Asn Gly Gly Cys Ala Pro Glu Ala Glu
            340                 345                 350
Cys Lys Met Thr Asp Ser Asn Lys Ile Val Cys Lys Cys Thr Lys Glu
        355                 360                 365
Gly Ser Glu Pro Leu Phe Glu Gly Val Phe Cys Ser
    370                 375                 380
(2) INFORMATIONS POUR LA SEQ ID NO: 14:
(i) CARACTERISTIQUES DE LA SEQUENCE:
(A) LONGUEUR: 281 acides aminEs
(B) TYPE: acide aminE
(C) NOMBRE DE BRINS: simple
(D) CONFIGURATION: linEaire
(ii) TYPE DE MOLECULE: peptide
(ix) CARACTERISTIQUE:
(A) NOM/CLE: Protein
(B) EMPLACEMENT:1__281
(D) AUTRES INFORMATIONS:/label= Consensus
/note= "FIGURE 4"
(ix) CARACTERISTIQUE:
(A) NOM/CLE: Region
(B) EMPLACEMENT:1__115
(D) AUTRES INFORMATIONS:/note= "REGION I"
(ix) CARACTERISTIQUE:
(A) NOM/CLE: Region
(B) EMPLACEMENT:116__125
(D) AUTRES INFORMATIONS:/note= "REGION II"
(ix) CARACTERISTIQUE:
(A) NOM/CLE: Region
(B) EMPLACEMENT:126__197
(D) AUTRES INFORMATIONS:/note= "REGION III"
(ix) CARACTERISTIQUE:
(A) NOM/CLE: Region

(B) EMPLACEMENT: 198__281
(D) AUTRES INFORMATIONS: /note= "REGION IV"
(xi) DESCRIPTION DE LA SEQUENCE: SEQ ID NO: 14:
Asp Gln Val Thr Thr Gly Glu Ala Glu Ser Glu Ala Pro Glu Ile Val
1               5                   10                  15
Pro Gly Ile Tyr Asp Val Val Tyr Lys Pro Leu Ala Gly Met Tyr Lys
            20                  25                  30
Thr Ile Lys Lys Leu Glu Asn His Val Asn Ala Asn Thr Asn Ile Asp
        35                  40                  45
Met Leu Asp Ser Ala Leu Lys Lys Ala Asn Tyr Phe Leu Val Leu Asn
    50                  55                  60
Ser Asp Leu Asn Pro Ser Gly Glu Tyr Ile Ile Lys Asp Pro Tyr Lys
65                  70                  75                  80
Leu Leu Asp Leu Glu Lys Lys Lys Leu Gly Ser Tyr Lys Tyr Ile Gly
                85                  90                  95
Ala Ser Asp Asp Thr Ala Asn Asp Gly Tyr Tyr Lys Met Gly Leu Tyr
            100                 105                 110
Lys His Leu Val Lys Val Glu Ile Asp Lys Lys Gly Lys Ala Lys Lys
        115                 120                 125
Glu Leu Lys Tyr Leu Pro Phe Leu Ser Gln Lys Glu Tyr Leu Val Lys
    130                 135                 140
Val Tyr Thr Asp Leu Lys Lys Ile Asn Asn Cys Gln Glu Lys Lys Glu
145                 150                 155                 160
Glu Val Lys Leu Asp Tyr Lys Met Asp Glu Leu Tyr Lys Ser Lys Val
                165                 170                 175
Lys Ser Ser Gly Leu Leu Glu Lys Leu Met Ser Lys Leu Ile Glu Ser
            180                 185                 190
Lys Leu Ser Leu Leu Asn Val Gln Thr Gln Leu Met Ser Ser Glu His
        195                 200                 205
Cys Ile Asp Thr Asn Val Pro Asn Ala Ala Cys Tyr Arg Tyr Leu Asp
    210                 215                 220
Gly Thr Glu Glu Trp Arg Cys Leu Leu Phe Lys Glu Gly Lys Cys Val
225                 230                 235                 240
Pro Ala Asn Thr Cys Lys Asp Asn Gly Gly Cys Ala Pro Glu Ala Glu
                245                 250                 255
Cys Lys Met Asp Asn Ile Val Cys Lys Cys Thr Lys Glu Gly Ser Glu
            260                 265                 270
Pro Leu Phe Glu Gly Val Phe Cys Ser
        275                 280

## Revendications

1. **Composition de vaccin** comprenant à titre de principes actifs un mélange de :
**a)** une protéine recombinante comprenant :
- un fragment C-terminal de 19 kilodaltons (p19) de la protéine 1 de surface (MSP-1) de la forme mérozoïte d'un *Plasmodium falciparum*, ce fragment C-terminal restant normalement ancré à la surface du parasite au terme de sa pénétration dans des érythrocytes humains, à l'occasion d'un cycle infectieux,
ou
- une partie dudit fragment p19 qui est apte à induire une réponse immune capable d'inhiber une parasitémie due à un parasite *Plasmodium falciparum*, et qui contient au moins l'une des deux régions EGF contenues dans ledit fragment p19,
ladite protéine
- comportant des épitopes conformationnels instables en milieu réducteur et constituant la majorité des épitopes reconnus par des antisérums humains formés contre *Plasmodium falciparum,* et
- étant reconnue par des antisérums humains formés contre *Plasmodium falciparum* lorsqu'elle est à l'état non réduit ou dans un état réduit non irréversible, mais non reconnue ou peu reconnue par ces mêmes antisérums, lorsqu'elle est irréversiblement réduite,
et de
**b)** une autre protéine recombinante p19 provenant d'un parasite *Plasmodium* homologue *à P. falciparum.*

2. Composition vaccinante selon la revendication 1, **caractérisée en ce que** ledit fragment p19 ou partie de fragment p19 qui est compris dans ladite protéine recombinante selon a) est :
i) susceptible d'être exprimé par un vecteur baculovirus recombinant contenant, sous le contrôle d'un promoteur contenu dans ce vecteur et susceptible d'être reconnu par des cellules transfectables par ce vecteur, un ADN qui comprend une séquence nucléotidique synthétique qui code ledit fragment p19 ou partie de fragment p19,
ladite séquence nucléotidique synthétique ayant en outre une teneur en G et C comprise entre 40 et 60%, de préférence d'au moins 50%, de la totalité des nucléotides dont elle est constituée,
ledit ADN comprenant en outre une séquence nucléotidique codant un peptide signal, en amont de l'extrémité 5'-terminale de ladite séquence nucléotidique synthétique, ce peptide signal étant susceptible d'être reconnu en tant que signal
dans un système baculovirus,
ou **en ce que** ledit fragment p19 ou partie de fragment p19 est :
ii) susceptible d'être produit par une cellule d'insecte transfectée par un vecteur baculovirus tel que défini en i).

3. Composition de vaccin selon la revendication 2, **caractérisée en ce que** ladite séquence nucléotidique synthétique est dépourvue à son extrémité 3'-terminale de la séquence codant pour la région d'extrémité C-terminale hydrophobe normalement impliquée dans l'induction de l'ancrage du fragment p19 à la membrane cellulaire de l'hôte dans lequel il est exprimé, notamment dans une cellule d'insecte infectable par un baculovirus.

4. Composition de vaccin selon la revendication 1 ou 2, **caractérisée en ce que** ladite protéine selon a) comporte également un groupe glycosylphosphatidylinositol (GPI) du type de ceux qui permettent un ancrage du fragment p19 à l'hôte cellulaire, notamment une cellule eucaryote, préférentiellement une cellule d'insecte infectable par un baculovirus, dans lequel ladite protéine recombinante est exprimée.

5. Composition de vaccin selon l'une quelconque des revendications 2 à 4, **caractérisée en ce que** ladite séquence nucléotidique synthétique est précédée d'une séquence nucléotidique signal codant pour un peptide signal normalement associé à une protéine MSP-1 de *Plasmodium,* homologue ou hétérologue vis-à-vis de ladite séquence nucléotidique synthétique.

6. Composition de vaccin selon la revendication 5, **caractérisée en ce que** ladite séquence signal est issue de *P. vivax.*

7. Composition de vaccin selon l'une quelconque des revendications 1 à 6, **caractérisée en ce que** ladite séquence signal est issue de *P. falciparum.*

8. Composition de vaccin selon l'une quelconque des revendications 2 à 7, **caractérisée en ce que** ledit ADN comprend en outre, en amont de ladite séquence nucléotidique synthétique, une séquence nucléotidique qui code une séquence polypeptidique comprenant moins de 50 résidus, notamment moins de 35 résidus, voire moins de 10 résidus de la partie C-terminale du fragment de 33 kilodaltons (p33) de MSP-1.

9. Composition de vaccin selon l'une quelconque des revendications 1 à 8, **caractérisée en ce que** ledit fragment p19 ou partie de fragment p19 qui est compris dans la protéine recombinante selon a) a un poids moléculaire qui est compris entre 10 et 25 kDa, notamment entre 10 et 15 kDa.

10. Composition de vaccin selon l'une quelconque des revendications 2, 3, 6,
**caractérisée en ce que** :
ladite séquence nucléotidique synthétique code un fragment C-terminal de 19 kilodaltons (p19) de la protéine 1 de surface (MSP-1) de la forme mérozoïte d'un *Plasmodium falciparum* de Asn1613 à Ser1705,
et **en ce qu'**en amont de l'extrémité 5'-terminale de cette séquence nucléotidique synthétique, l'ADN comprend:
- les 8 paires de bases de la séquence leader de la protéine 1 de surface de *Plasmodium vivax,* et
- une séquence codant les trente-deux acides aminés de la protéine 1 de surface de *Plasmodium vivax* de Met1 à Asp32,
et **en ce qu'**en aval de l'extrémité 3'-terminale de cette séquence nucléotidique synthétique, l'ADN comprend deux codons stop TAA.

11. Composition de vaccin selon l'une quelconque des revendications 2, 4, 6,
**caractérisée en ce que** :
ladite séquence nucléotidique synthétique code un fragment C-terminal de 19 kilodaltons (p19) de la protéine 1 de surface (MSP-1) de la forme mérozoïte d'un *Plasmodium falciparum* de Asn1613 à Ile1726,
et **en ce qu'**en amont de l'extrémité 5'-terminale de cette séquence nucléotidique synthétique, l'ADN comprend:
- les 8 paires de bases de la séquence leader de la protéine 1 de surface de *Plasmodium vivax,* et
- une séquence codant les trente-deux acides aminés de la protéine 1 de surface de *Plasmodium vivax* de Met1 à Asp32,
et **en ce qu'**en aval de l'extrémité 3'-terminale de cette séquence nucléotidique synthétique, l'ADN comprend deux codons stop TAA.

12. Composition de vaccin selon l'une quelconque des revendications 1 à 11, **caractérisée en ce que** ledit vecteur baculovirus selon i) est le virus déposé à la C.N.C.M. sous le numéro I-1661, ou le virus déposé à la C.N.C.M. sous le numéro I-1662.

13. Composition de vaccin selon l'une quelconque des revendications 1 à 12, **caractérisée en ce que** ladite protéine selon a) est conjuguée à une molécule porteuse utilisable pour la production de vaccins.

14. Composition de vaccin selon l'une quelconque des revendications 1 à 13, **caractérisée en ce que** ladite protéine recombinante selon b) est la p19 de *Plasmodium vivax*.

15. Composition de vaccin selon l'une quelconque des revendications 1 à 14, **caractérisée en ce qu'**en outre de ladite protéine recombinante selon a), elle comprend :
- la p19 de *P. vivax,*
- la p42 de *P. falciparum,* ladite p42 étant le cas échéant délétée de ses régions les plus hypervariables, et
- la p42 de *P. vivax,* ladite p42 étant le cas échéant délétée de ses régions les plus hypervariables.
